# EUROPEAN PATENT APPLICATION

(11) **EP 4 230 214 A1**
(43) Date of publication of application: **23.08.2023**
(21) Application number: 21886520.2
(22) Date of filing: 27.08.2021
(51) Int. Cl.: A61K 36/28, A61K 31/365, A61K 9/00, A61P 37/00

(54) **COMPOSITION FOR PREVENTING AND TREATING ANTI-INFLAMMATORY AND AUTOIMMUNE DISEASES AND NON-ALCOHOLIC FATTY LIVER DISEASE, COMPRISING EXTRACT DERIVED FROM CENTIPEDA MINIMA**

(30) Priority: 30.10.2020 KR 20200142792
(71) Applicant: D-Nature Co., Ltd., Seongnam-si, Gyeonggi-do 13174 (KR)
(72) Inventor: KIM, Byoungha, Hanam-si, Gyeonggi-do 13013 (KR); YUN, Sangkyu, Hwaseong-si, Gyeonggi-do 18472 (KR); KIM, Cheolsun, Seongnam-si, Gyeonggi-do 13183 (KR)
(74) Representative: BCKIP Part mbB
(86) International application number: PCT/KR2021/011534
(87) International publication number: WO 2022/092523

(57) **Abstract**

Disclosed is a composition for preventing and treating anti-inflammatory and autoimmune diseases and non-alcoholic fatty liver disease. A composition for preventing and treating anti-inflammatory and autoimmune diseases and non-alcoholic fatty liver disease according to an embodiment of the present invention includes an extract of centipeda minima comprising, as an active ingredient, at least one selected from the group consisting of Brebilin A, Arnicolide D, Arnicolide C, and Microhelenin C.

## Description

### TECHNICAL FIELD

The present invention relates to a composition for preventing and treating inflammatory disease and autoimmune diseases and non-alcoholic fatty liver disease, and more specifically, as an extract derived from *Centipeda minima,* a composition for preventing and treating inflammatory disease and autoimmune diseases and non-alcoholic fatty liver disease, comprising Brevilin A, Arnicolide D, Arnicolide C, and Microhelenin C as active ingredients.

### BACKGROUND ART

Autoimmune diseases, which are diseases caused by immune cells attacking organs or tissues of their own body, are incurred by immune imbalance or loss of resistance to autoantigens. Autoimmune diseases cause damage to cells or tissues by humoral immunity, cellular immunity, or both. Such an inappropriate response to autoantigens of the immune system is referred to as autoimmunity. Autoimmune diseases are associated with numerous molecules, cells, and tissues targeted by autoimmune responses, and may be systemic or specific to a certain organ depending on the distribution of the target antigens. For example, systemic lupus erythematosus (SLE), rheumatoid arthritis (RA), and multiple sclerosis (MS) are systemic autoimmune diseases. In addition, alopecia areata is an autoimmune disease that shows uneven hair loss, regardless of gender, age, and hair color worldwide, and alopecia areata exhibits a prevalence of about 0.1 to 0.2% worldwide.

Tofacitinib is widely used as an immunosuppressant for autoimmune diseases.

Tofacitinib, {3-((3R,4R)-4-methyl-3-(methyl(7H-pyrrolo[2,3-d]pyrimidin-4-yl)amino)piperidin-1-yl)-3-oxopropanenitrile}, is a compound represented by the formula shown below. The tofacitinib was first disclosed in WO 2001/042246.

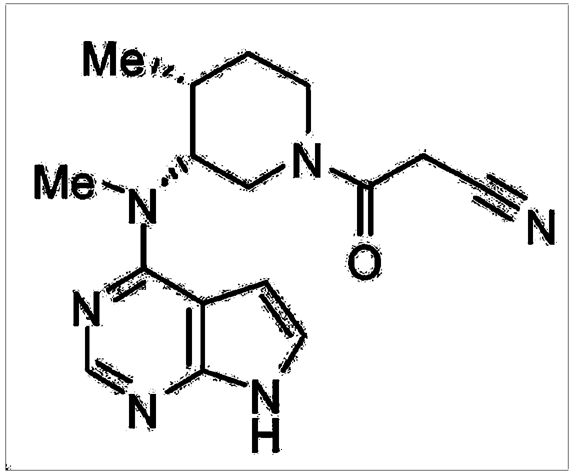

Tofacitinib is a synthetic JAK (janus kinase) inhibitor, and janus kinase (JAK) is one of the enzymes that catalyze the transfer of a phosphate group. Cytokine, a protein secreted by immune cells, binds to a cytokine receptor and transmits a signal into a cell. Cytokine receptors bind to janus kinases such as TYK2, JAK1, JAK2, and JAK3.

When a cytokine binds to a cytokine receptor, phosphorylation occurs in JAK, the signal transducer and activator of transcription (STAT) is activated, and then signaling occurs, leading to hematopoietic action, inflammatory response, and immune response. In an autoimmune disease in which a hyperimmune response occurs due to cytokine overproduction, a JAK inhibitor inhibits the kinase of the cytokine receptor to which the cytokine binds.

However, not only tofacitinib but also JAKI and JAK inhibitors of the same class are synthetic drugs of which safety may not be guaranteed due to the numerous side effects identified from their raw materials. In addition, developed as prescription drugs, they have low patient accessibility, and their use is limited to pharmaceuticals for oral administration. Furthermore, their long-term use causes serious infections (nasopharyngitis, gastroenteritis, upper respiratory tract infection) and intestinal perforation due to immunosuppression, and elevated cholesterol, headache, rash, herpes zoster, anemia, and the like are caused. Moreover, based on the daily prescription of 10 mg/day, tofacitinib causes a high cost of about 6 million KRW per month when domestic health insurance does not apply.

Fatty liver is caused by the accumulation of excessive fat, mainly triglycerides, in the liver. In general, fat accumulation of more than 5% of the liver's weight is diagnosed as fatty liver. Fatty liver can be divided into alcoholic fatty liver and non-alcoholic fatty liver (NASH), which is caused in association with obesity, diabetes, and hyperlipidemia. Non-alcoholic fatty liver is associated with metabolic syndrome such as obesity, adult-onset diabetes, and hyperlipidemia. When excessive caloric intake continues, fat is accumulated in the adipocytes and the liver of the body, and various substances (cytokines) are secreted from the increased fat to cause fatty hepatitis and cirrhosis. In addition, drugs such as steroids, anti-inflammatory drugs, and heart drugs or some herbal medicines or several folk remedies can also cause fatty liver.

*Centipeda minima* is an annual plant belonging to the *Asteraceae* family. In China, the plant is used in folk remedies to treat chronic rhinitis as well as nose diseases, eye diseases, and headache. In addition, when the leaves and stems are rubbed and placed in the nostrils and kept overnight, they are effective against chronic malaria. In India, the plant is used for eye diseases, nose diseases, and toothache. The plant is distributed in Korea, Japan, China, India, Australia, eastern Siberia, and North America. The efficacy of *Centipeda minima* as a raw material on inflammatory disease and autoimmune diseases, and non-alcoholic fatty liver disease is not known, and so research is required.

Natural active ingredients are extracted and selected from natural raw materials. As a general extraction method, when the raw material is a solid, a solid-liquid extraction method is used, when the raw material is a liquid, a liquid-liquid extraction (LLE) method is used. Liquid-liquid extraction is the separation of certain components of a mixture from other components by the action of a solvent on a liquid mixture containing a solute.

In general, a liquid-liquid extraction method is used. During liquid-liquid extraction, non-polar solvents such as hexane, chloroform (methane dichloride), and ethyl acetate are highly toxic. The method has problems that the extraction process is highly inefficient, complicated, and long because of the several repeated extraction processes and the drying process for removing non-polar solvents, and the production cost is high due to the low extraction efficiency and long process.

For example, in the process for extracting Brevilin A from *Centipeda minima,* the raw material is extracted with methanol multiple times; the resulting extracted liquid is mixed with hexane and fractionated multiple times; the resulting fraction is mixed with chloroform and fractionated multiple times; the resulting fraction is mixed with ethyl acetate and fractionated multiple times; and the resulting fraction is mixed with butyl alcohol and fractionated multiple times. Since about 25 mg of Brevilin A is extracted from 300 g of dry weight of *Centipeda minima,* the extraction amount is extremely little and the efficiency is very low (Non-Patent Document 1).

Therefore, an optimal extraction method capable of maximizing the extraction amount of the active ingredient of a tiny amount is required. The active ingredient can be extracted by the extraction method of Korean Patent Publication No. 10-2020-0085024, which is an application of the present applicant.

Therefore, while studying a composition for preventing and treating inflammatory disease and autoimmune diseases and non-alcoholic fatty liver disease, the present inventors found that a composition extracted by the optimal extraction method, as an extraction of *Centipeda minima,* comprising Brevilin A, Arnicolide D, Arnicolide C, and Microhelenin C as active ingredients, is effective on inflammatory disease and autoimmune diseases and non-alcoholic fatty liver disease with few side effects.

Patent Document 1: Korean Patent Publication No. 10-2020-0085024.

Non-Patent Document 1: Arch Pharm Res Vol 29, No 1, 64-66, 2006.

### DETAILED DESCRIPTION OF THE INVENTION

### TECHNICAL PROBLEM

Examples of the present invention provide, as an extract derived from *Centipeda minima,* a composition for preventing and treating inflammatory disease and autoimmune diseases and non-alcoholic fatty liver disease, comprising Brevilin A, Arnicolide D, Arnicolide C, and Microhelenin C as active ingredients.

### TECHNICAL SOLUTION

A composition for preventing and treating inflammatory disease and autoimmune diseases and non-alcoholic fatty liver disease according to an Example of the present invention comprises a *Centipeda minima* extract comprising at least one selected from the group consisting of Brevilin A, Arnicolide D, Arnicolide C, and Microhelenin C of the formulas below as an active ingredient.

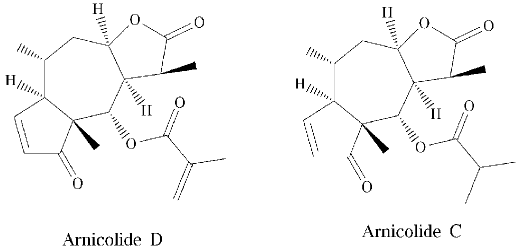

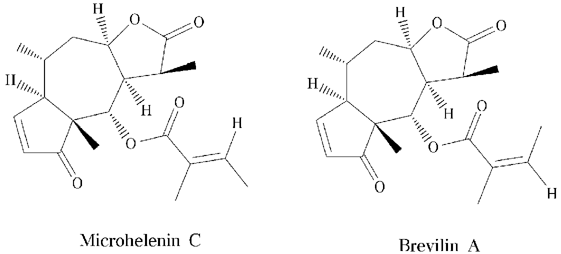

In addition, according to one Example of the present invention, provided is a composition for preventing and treating inflammatory disease and autoimmune diseases and non-alcoholic fatty liver disease, wherein the Brevilin A inhibits or suppresses JAK-STAT signaling process; the Arnicolide D, the Arnicolide C, and the Microhelenin C inhibit or suppress cytokine-cytokine receptor interaction, MAPK signaling pathway, and PI3K-AKT signaling pathway; and the *Centipeda minima* extract activates WNT signaling pathway (WNT/β-catenin pathway).

In addition, according to one Example of the present invention, provided is a composition for preventing and treating inflammatory disease and autoimmune diseases and non-alcoholic fatty liver disease, wherein the content of the Brevilin A is more than double the total content of the Arnicolide D, the Arnicolide C, and the Microhelenin C.

In addition, according to one Example of the present invention, provided is a composition for preventing and treating inflammatory disease and autoimmune diseases and non-alcoholic fatty liver disease, wherein the *Centipeda minima* extract comprises 30 to 90% by weight of the Brevilin A; 0 to 30% by weight of the Arnicolide D; 0 to 30% by weight of the Arnicolide C; and 0 to 15% by weight of the Microhelenin C.

In addition, according to one Example of the present invention, provided is a composition for preventing and treating inflammatory disease and autoimmune diseases and non-alcoholic fatty liver disease, wherein the *Centipeda minima* extract is extracted by an extraction method comprising: preparing a primary extraction by extracting a *Centipeda minima* raw material; performing secondary extraction by mixing a phase separation composition comprising a lipophilic solubilizer and water with the primary extraction; and obtaining a non-polar natural substance by separating a top layer of a phase-separated solution.

In addition, according to one Example of the present invention, provided is a composition for preventing and treating inflammatory disease and autoimmune diseases and non-alcoholic fatty liver disease, wherein the composition for preventing and treating inflammatory disease and autoimmune diseases and non-alcoholic fatty liver disease further comprises at least one selected from the group consisting of a lipophilic solubilizer, a surfactant, and water.

In addition, according to one Example of the present invention, provided is a composition for preventing and treating inflammatory disease and autoimmune diseases and non-alcoholic fatty liver disease, wherein the lipophilic solubilizer comprises at least one selected from the group consisting of BRIJ 010-SS-(RB), Capryol 90, Capryol PGMC, Gantrez ES-435, Gantrez S-97 BF, Gelucire 43/01, Gelucire 44/14, Gelucire 50/13, Gelucire 48/16, Labrafac CC, Labrafac Lipophile WL1349, Labrafac PG, Labrafil M 1944 CS, Labrafil M 2125 CS, Labrafil M 2130 CS, Lauroglycol 90, Lauroglycol FCC, Monosteol, Peceol, Pharmasolve, Plurol Oleique CC497, Span 20-LQ-(SG), Span 60-PA-(SG), upper Refined Oleic Acid-LQ-(JP), Super Refined Tween 80A-LQ-(MH), Surfadone LP-300, Transcutol HP, Transcutol P, TWEEN 60-SS-(SG), TWEEN 80 HP-LQ-(MH), TWEEN 80-LQ-(SG), Vitamin E TPGS, Miglyol 812, and captax 355.

In addition, according to one Example of the present invention, provided is a composition for preventing and treating inflammatory disease and autoimmune diseases and non-alcoholic fatty liver disease, wherein the surfactant comprises at least one selected from the group consisting of Labrasol, Crodex A-PA-(RB), Geleol, Gelot 64, Suppocire AS2X, Suppocire BML, Synperonic PE/F 127, Synperonic PE/L 44, and Tefose 63.

In addition, according to one Example of the present invention, provided is a composition for preventing and treating inflammatory disease and autoimmune diseases and non-alcoholic fatty liver disease, wherein the inflammatory disease and autoimmune diseases and non-alcoholic fatty liver disease comprise at least one selected from the group consisting of hair loss, alopecia areata, rheumatoid arthritis, psoriasis, atopy, eczema, seborrheic dermatitis, scleroderma, skin immune hypersensitivity, multiple sclerosis, lupus, Behçet's disease, ankylosing spondylitis, Sjogren's syndrome, Crohn's disease, and inflammatory bowel disease.

A preventive and therapeutic agent of inflammatory disease and autoimmune diseases and non-alcoholic fatty liver disease according to another Example of the present invention comprises the composition for preventing and treating inflammatory disease and autoimmune diseases and non-alcoholic fatty liver disease.

In addition, according to one Example of the present invention, provided is a preventive and therapeutic agent of inflammatory disease and autoimmune diseases and non-alcoholic fatty liver disease, wherein the composition for preventing and treating inflammatory disease and autoimmune diseases and non-alcoholic fatty liver disease comprises 0.1 to 90% by weight of the extract of the *Centipeda minima* extract based on the total weight of the composition.

### ADVANTAGEOUS EFFECTS

Examples of the present invention, directed to a composition for preventing and treating inflammatory disease and autoimmune diseases and non-alcoholic fatty liver disease, use a natural extract in contrast to tofacitinib, which is an existing synthetic drug for treating autoimmune diseases, thus ensuring the safety of the raw material. Moreover, compared to the single prescription of Brevilin A, which is known as an extract of *Centipeda minima,* the composition exhibits a better effect on hair growth and hair loss prevention without a side effect.

In addition, Examples of the present invention, directed to a composition for preventing and treating non-alcoholic fatty liver disease, have an effect of preventing inflammation and fibrosis in hepatocytes through the inhibition of adipocyte differentiation and insulin resistance.

In addition, while existing synthetic drugs are limited to oral administration, the Examples of the present invention using a natural composition have little or no side effects compared to synthetic drugs, and can easily be applied to cosmetics and foods as well as pharmaceuticals.

### DESCRIPTION OF THE DRAWINGS

FIGS. 1 to 3 are diagrams analyzing active ingredients of a *Centipeda minima* extract according to an Example of the present invention, wherein FIG. 1 is a graph representing an HPLC analysis of the ingredients contained in *Centipeda minima* extract according to an Example of the present invention; FIG. 2 is a graph representing an HPLC analysis of a mixture of Brevilin A, Arnicolide D, Arnicolide C, and Microhelenin C for the analysis shown in FIG. 1; and FIG. 3 shows formulas of Brevilin A, Arnicolide D, Arnicolide C, and Microhelenin C.
FIGS. 4 to 6 are diagrams showing the UHPLC-Q-TOF mass spectrum for analyzing the individual ingredients of a *Centipeda minima* extract according to an Example of the present invention.
FIG. 7 shows the changing pattern and improvement rate of the average total hair counts (N/cm²) of the test group and the control group, as the results of an experiment performed to confirm the hair growth and hair loss prevention effects of a composition for preventing and treating inflammatory disease and autoimmune diseases according to an Example of the present invention.
FIGS. 8 and 9 are graphs showing the changing pattern and improvement rate of the average total hair counts (N/cm²) of the test group and the control group according to the experimental results shown in FIG. 7.
FIG. 10 shows the average score of the tester's visual evaluation representing the degree of hair improvement in the test group and the control group according to the experimental results shown in FIG. 7.
FIG. 11 is a graph showing the average score of the tester's visual evaluation representing the degree of hair improvement in the test group and the control group according to the experimental results shown in FIG. 10.
FIGS. 12 to 15 show the contents of inflammation-related factors Ccl2, IL-1b, TNF-a, and IL-10 to confirm the inflammatory disease effect of a composition for preventing and treating inflammatory disease and autoimmune diseases according to an Example of the present invention.
FIGS. 16 to 21 show the contents of inflammation-related factors IL-1b, IL-6, TNF-a to confirm the inflammatory disease effect of a composition for preventing and treating inflammatory disease and autoimmune diseases according to an Example of the present invention.
FIG. 22 is an illustration confirming the effect of inhibiting adipocyte differentiation by a composition for preventing and treating inflammatory disease and autoimmune diseases and non-alcoholic fatty liver disease according to an Example and a control group of the present invention.
FIG. 23 is an illustration confirming the cytotoxicity to hepatocytes by a composition for preventing and treating inflammatory disease and autoimmune diseases and non-alcoholic fatty liver disease according to an Example and a control group of the present invention.
FIG. 24 is an illustration confirming the effect of a composition for preventing and treating inflammatory disease and autoimmune diseases and non-alcoholic fatty liver disease according to an Example and a control group of the present invention on TNF-α-induced IL-6 production.
FIG. 25 is an illustration confirming the effect of a composition for preventing and treating inflammatory disease and autoimmune diseases and non-alcoholic fatty liver disease according to an Example and a control group of the present invention on TNF-α-induced MCP-1 production.
FIGS. 26 to 28 are illustrations confirming the cell viability of hepatic stellate cells and protein expression changes related to inhibition of hepatic fibrosis by a composition for preventing and treating inflammatory disease and autoimmune diseases and non-alcoholic fatty liver disease according to an Example of the present invention.
FIG. 29 is an illustration confirming protein expression changes related to inhibition of hepatic fibrosis according to a control group of the present invention.

### BEST MODE

A composition for preventing and treating inflammatory disease and autoimmune diseases and non-alcoholic fatty liver disease according to an Example of the present invention comprises a *Centipeda minima* extract comprising at least one selected from the group consisting of Brevilin A, Arnicolide D, Arnicolide C, and Microhelenin C of the formulas below as an active ingredient.

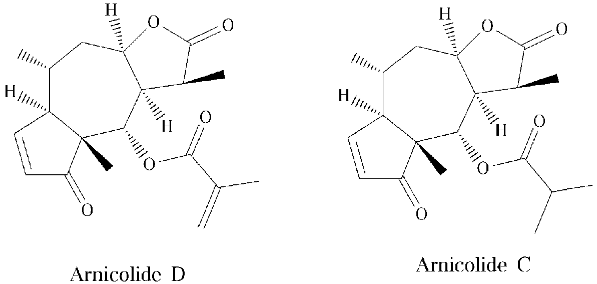

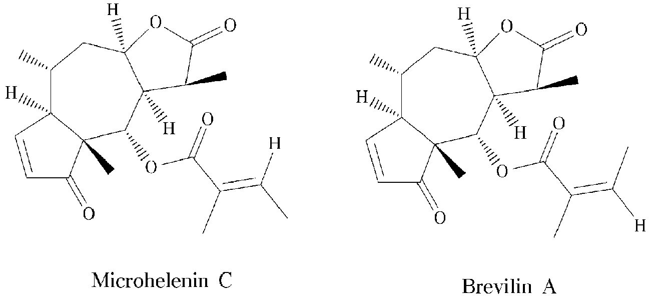

### MODE OF INVENTION

Hereinafter, a method for extracting non-polar natural substances according to Examples of the present invention, natural substances extracted according to the method, and uses thereof will be described.

Before that, technical terms used in this specification are only for referring to specific Examples and are not intended to limit the present invention. In addition, singular forms used herein also include plural forms, unless the phrases clearly indicate the opposite. In addition, the meaning of 'comprising' or 'containing' as used herein specifies a specific characteristic, region, integer, step, operation, element, or ingredient, and addition of other specific characteristics, regions, integers, steps, operations, elements, or ingredients is not excluded.

In the present invention, terms such as 'first' and 'second' are used to describe various components, and the terms are used only for the purpose of distinguishing one component from another.

In addition, terms used in this specification are only used to describe illustrative Examples, and are not intended to limit the present invention. Singular expressions include plural expressions unless the context clearly dictates otherwise. In this specification, terms such as 'comprise,' `provided with' or 'having' are intended to indicate that there is an implemented feature, number, step, component, or combination thereof, and they should be understood as not precluding the possibility of the presence or addition of one or more other features or numbers, steps, components, or combinations thereof.

Since the present invention can have various changes and various forms, specific Examples will be exemplified and described in detail below. However, it should be understood that this is not intended to limit the present invention to the specific disclosed form, and includes all modifications, equivalents, and substitutes included in the principle and scope of the present invention.

Hereinafter, the present invention will be described in more detail.

A composition for preventing and treating inflammatory disease and autoimmune diseases and non-alcoholic fatty liver disease according to an Example of the present invention comprises a *Centipeda minima* extract.

Here, the autoimmune disease is a disease caused by an abnormal immune response due to autoimmune, but is not limited thereto, and may be, for example, hair loss, alopecia areata, rheumatoid arthritis, psoriasis, atopy, eczema, seborrheic dermatitis, scleroderma, skin immune hypersensitivity, multiple sclerosis, lupus, Behçet's disease, ankylosing spondylitis, Sjögren's syndrome, Crohn's disease, and inflammatory bowel disease.

The extract *Centipeda minima* extract comprises at least one selected from the group consisting of Brevilin A, Arnicolide D, Arnicolide C, and Microhelenin C as an active ingredient.

Brevilin A is represented by the formula below.

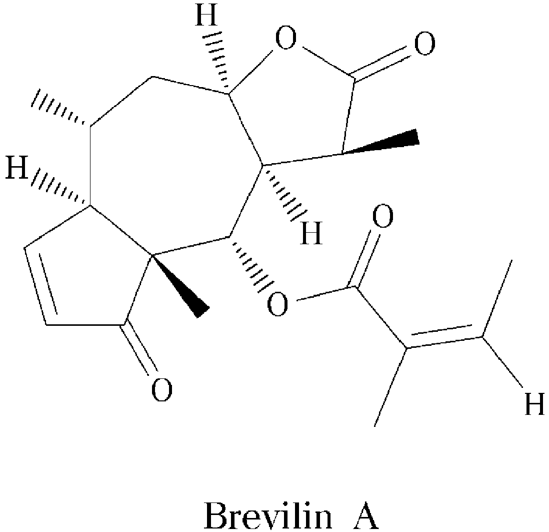

Brevilin A, having CAS number 16503-32-5, a molecular formula of C₂₀H₂₆O₅, and a molecular weight of 346.42, is derived from *Arnica* sp., *Centipeda* sp., and *Helenium* sp.

A number of documents indicating that Brevilin A is contained in *Centipeda minima* (also referred to as 'Abulsikcho') have been found, and some documents reported that Brevilin A is also contained in *Litsea glutinous.* In addition, documents indicating that a certain amount of Brevilin A is contained in plants of the *Arnica* genus (*Arnica longifolia, Arctium lappa, Arnica chamissonis* subsp. Foliosa, and *Arnica chamissonis* Less.) and plants of the *Helenium* genus (*Helenium autumnale* L, *Helenium alternifolium, Helenium pinnatifidum, Helenium vernale,* Helenium brevifolium, and *Helenium flexuosum*) have been found.

Arnicolide D is represented by the formula below.

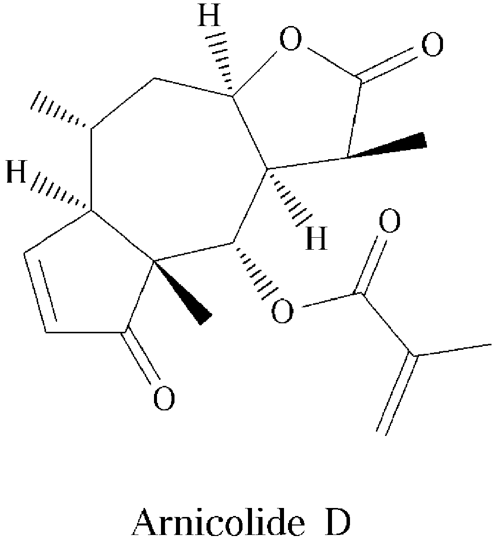

Arnicolide D, having CAS number 34532-68-8, a molecular formula of C₁₉H₂₄O₅, and a molecular weight is 332.40, is derived from *Arnica* sp. and *Centipeda* sp.

Arnicolide C is represented by the formula below.

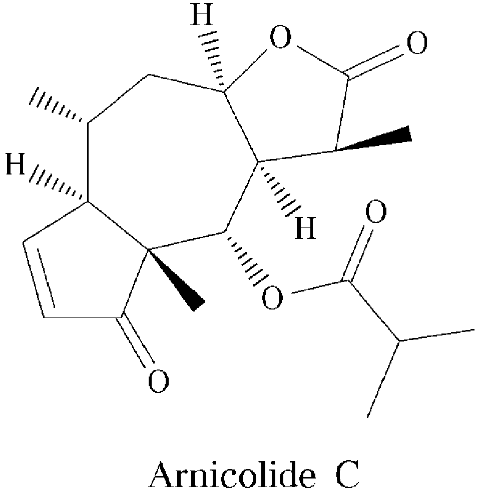

Arnicolide C, having CAS number 34532-67-7, a molecular formula of C₁₉H₂₆O₅, and a molecular weight is 334.41, is derived from *Arnica* sp. and *Centipeda* sp.

Microhelenin C is represented by the formula below.

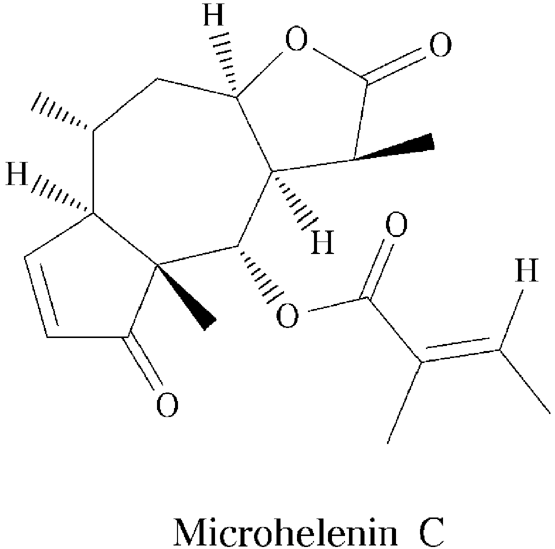

Microhelenin C, having CAS number 63569-07-3, a molecular formula of C₂₀H₂₆O₅, and a molecular weight is 346.4, is derived from *Centipeda* sp. and *Helenium* sp.

A composition for preventing and treating inflammatory disease and autoimmune diseases and non-alcoholic fatty liver disease according to an Example of the present invention comprises a *Centipeda minima* extract, wherein the *Centipeda minima* extract comprises at least one of Brevilin A, Arnicolide D, Arnicolide C, and Microhelenin C, for example, comprising each of Brevilin A, Arnicolide D, Arnicolide C, and Microhelenin C alone, or comprising a combination of Brevilin A and Arnicolide D, a combination of Brevilin A and Arnicolide C, a combination of Brevilin A and Microhelenin C, a combination of Arnicolide D and Arnicolide C, a combination of Arnicolide D and Microhelenin C, a combination of Arnicolide C and Microhelenin C, a combination of Brevilin A, Arnicolide D, and Arnicolide C, a combination of Brevilin A, Arnicolide D, and Microhelenin C, a combination of Arnicolide D, Arnicolide C, and Microhelenin C, or a combination of Brevilin A, Arnicolide D, Arnicolide C, and Microhelenin C.

The action mechanism of each of the Brevilin A, the Arnicolide D, the Arnicolide C, and the Microhelenin C is described below.

The Brevilin A inhibits or suppresses the JAK-STAT signaling pathway.

The Brevilin A, which is a natural substance, is widely known to inhibit janus kinase activity (JAK) and suppresses STAT3 signaling in cancer cells (PLoS One 8 (5) (2013*) e63697*).

The Arnicolide D, the Arnicolide C, and the Microhelenin C inhibit or suppress cytokine-cytokine receptor interaction, mitogen-activated protein kinase (MAPK) signaling pathway, and phosphatidylinositol-3-kinase (PI3K)-AKT signaling pathway (Hair Growth Effect of Emulsion Extracted Brevilin A, a JAK3 Inhibitor, from Centipeda minima, Processes 2020, 8, 767; doi :10.3390/pr8070767)*.*

The most ideal drug candidates for NASH should reduce hepatic inflammation and hepatocellular damage, correct insulin resistance, and at the same time, have antifibrotic effects. Failure to simultaneously control these various targets can cause problems such as fibrosis. The composition is one of the ideal drug candidates that can correct insulin resistance and suppress adipocyte differentiation and fibrosis without liver damage, and provides a composition for preventing and treating fatty liver and non-alcoholic fatty liver disease. (Current efforts and trends in the treatment of NASH, Journal of Hepatology 2015 vol. 62 j S65-S75).

The *Centipeda minima* extract activates the WNT signaling pathway (WNT/β-catenin pathway) and facilitates the differentiation and proliferation of hair follicle stem cells and cells involved in hair growth (primary hair cells), and thus is effective for hair loss and alopecia areata among autoimmune diseases. The *Centipeda minima* extract increases the expression of WNT receptors and activates the WNT signaling pathway to enhance GSK3β phosphorylation and β-catenin accumulation, and activates extracellular signal-regulated kinase (ERK) (phosphorylation) to enhance the expression of growth factors such as VEGF and IGF-1 (Hair Growth Stimulation Effect of Centipeda minima Extract: Identification of Active Compounds and Anagen-Activating Signaling Pathways, Biomolecules 2021, 11, 976. https://doi.org/ 10.3390/biom11070976).

In addition, based on the powerful anti-inflammatory effect, the *Centipeda minima* has an effect of suppressing adipocyte differentiation, suppressing hepatic fibrosis in hepatic stellate cells (LX-2) (anti-fibrosis), suppressing insulin resistance, and preventing obesity.

The content of the Brevilin A may be more than double the total content of the Arnicolide D, the Arnicolide C, and the Microhelenin C.

The Brevilin A is the most important ingredient for inflammatory disease and autoimmune diseases and non-alcoholic fatty liver disease, for example, hair growth and hair loss, and anti-inflammation, and the content of Brevilin A is preferably more than the total content of the Arnicolide D, the Arnicolide C, and the Microhelenin C, which are other derivatives. The may ingredient of the non-polar natural substances of *Centipeda minima,* which is the raw material, is the Brevilin A.

The Brevilin A is most involved in JAK-STAT signaling process, which is the main action mechanism of inflammatory disease and autoimmune diseases and non-alcoholic fatty liver disease. When Brevilin A functions with its other derivatives, the Arnicolide D, the Arnicolide, C and the Microhelenin C, a synergic effect is caused on inflammatory disease and autoimmune diseases and non-alcoholic fatty liver disease to provide a better effect on inflammatory disease and autoimmune diseases and non-alcoholic fatty liver disease, for example, hair loss prevention and anti-inflammation, than in the case where each ingredient is contained alone.

For example, the *Centipeda minima* extract contains 30 to 90% by weight of the Brevilin A, 0 to 30% by weight of the Arnicolide D, 0 to 30% by weight of the Arnicolide C, and 0 to 15% by weight of the Microhelenin C.

The Brevilin A is included in an amount of 30 to 90% by weight, and when the amount is less than 30% or more than 90% by weight, the ingredient that is most related to the JAK-STAT signaling process becomes small and so the anti-inflammatory effect is lowered and the synergic actions with other ingredients are reduced, resulting in difficulties in sufficiently preventing and treating inflammatory disease and autoimmune diseases and non-alcoholic fatty liver disease.

The Arnicolide D is included in an amount of 0 to 30% by weight, and when the amount is more than 30% by weight, the synergic actions with other ingredients, which are Brevilin A, Arnicolide C, and Microhelenin C, are reduced, resulting in difficulties in sufficiently preventing and treating inflammatory disease and autoimmune diseases and non-alcoholic fatty liver disease. Preferably, the Arnicolide D is included in an amount of 15 to 30% by weight.

The Arnicolide C is included in an amount of 0 to 30% by weight, and when the amount is more than 30% by weight, the synergic actions with other ingredients, which are Brevilin A, Arnicolide D, and Microhelenin C, are reduced, resulting in difficulties in sufficiently preventing and treating inflammatory disease and autoimmune diseases and non-alcoholic fatty liver disease. Preferably, the Arnicolide D is included in an amount of 4 to 30% by weight.

The Microhelenin C is included in an amount of 0 to 15% by weight, and when the amount is more than 15% by weight, the synergic actions with other ingredients, which are Brevilin A, Arnicolide D, and Arnicolide C, are reduced, resulting in difficulties in sufficiently preventing and treating inflammatory disease and autoimmune diseases and non-alcoholic fatty liver disease. Preferably, the Microhelenin C is included in an amount of 3 to 15% by weight.

Therefore, when the content of each of the ingredients of the Brevilin A, the Arnicolide D, the Arnicolide C, and the Microhelenin C is out of the range, an anti-inflammatory efficacy may not be achieved.

According to one Example of the present invention, provided is a composition for preventing and treating inflammatory disease and autoimmune diseases and non-alcoholic fatty liver disease, wherein the *Centipeda minima* extract is extracted by an extraction method comprising: preparing a primary extraction by extracting a *Centipeda minima* raw material; performing secondary extraction by mixing a phase separation composition comprising a lipophilic solubilizer and water with the primary extraction; and obtaining a non-polar natural substance by separating a top layer of a phase-separated solution.

The extraction method of the present invention refers to a dissolution-emulsion extract (DEE) for non-polar natural substances. According to DEE, non-polar natural substances in a natural raw material are extracted and mixed with a phase separation composition to be dissolved and emulsified, and then the non-polar natural substances are extracted by phase separation.

The composition for preventing and treating inflammatory disease and autoimmune diseases and non-alcoholic fatty liver disease may further comprise at least one selected from the group consisting of a lipophilic solubilizer, a surfactant, and water.

For example, the lipophilic solubilizer may comprise at least one selected from the group consisting of BRIJ 010-SS-(RB), Capryol 90, Capryol PGMC, Gantrez ES-435, Gantrez S-97 BF, Gelucire 43/01, Gelucire 44/14, Gelucire 50/13, Gelucire 48/16, Labrafac CC, Labrafac Lipophile WL1349, Labrafac PG, Labrafil M 1944 CS, Labrafil M 2125 CS, Labrafil M 2130 CS, Lauroglycol 90, Lauroglycol FCC, Monosteol, Peceol, Pharmasolve, Plurol Oleique CC497, Span 20-LQ-(SG), Span 60-PA-(SG), upper Refined Oleic Acid-LQ-(JP), Super Refined Tween 80A-LQ-(MH), Surfadone LP-300, Transcutol HP, Transcutol P, TWEEN 60-SS-(SG), TWEEN 80 HP-LQ-(MH), TWEEN 80-LQ-(SG), Vitamin E TPGS, Miglyol 812, and captax 355. Miglyol 812 and captax 355, which are medium chain triglyceride (MCT) oils, may be used as the lipophilic solubilizer.

For example, as a non-ionic surfactant, the surfactant may comprise at least one selected from the group consisting of Labrasol, Crodex A-PA-(RB), Geleol, Gelot 64, Suppocire AS2X, Suppocire BML, Synperonic PE/F 127, Synperonic PE/L 44, and Tefose 63.

The extraction method is described in detail as below.

First, a primary extract is prepared by extracting natural substances from a *Centipeda minima* raw material. As an extraction solvent, various solvents such as water and alcohol may be selected and used, and alcohol was used in the present invention. Preferably, methanol or ethanol can be used.

For example, extraction may be performed by selecting any one of methods such as hot water extraction, cold extraction, reflux cooling extraction, solvent extraction, steam distillation, ultrasonic extraction, and elution, and additionally, a conventional fractionation process may be performed.

For example, cold extraction can be performed by immersing a natural raw material in cold alcohol. For example, as a natural raw material, *Centipeda minima* itself may be used, or a powder obtained by pulverizing the same may be used.

Unlike this, the extraction can be performed by hot water extraction in which a *Centipeda minima* raw material is immersed in alcohol at a temperature of 100 °C or higher for about 1 hour, and about 80% alcohol can be used. For example, a step of removing an alcohol component by concentrating under reduced pressure a primary extract according to hot water extraction may be performed. Although there is no difference in achieving the present invention even if a primary extract that has not undergone a concentrating step is used to directly move on to the next step, an unnecessary alcohol component may preferably be removed in consideration of clearer phase separation and extraction efficiency.

A phase separation composition is added to the primary extract and mixed.

The phase separation composition may comprise a lipophilic solubilizer and water. For example, the phase separation composition may comprise 10 to 90% by weight of a lipophilic solubilizer and 10 to 90% by weight of water.

For example, the phase separation composition may further comprise a nonionic surfactant. The phase separation composition may comprise 40% by weight or less of a nonionic surfactant.

Therefore, the Brevilin A, the Arnicolide D, the Arnicolide C, and the Microhelenin C contained in a *Centipeda minima* raw material are primarily extracted as non-polar natural substances, and then mixed with a phase separation composition. Phase separation occurs as a lipophilic solubilizer and non-polar natural substances are contained in one layer, and the remaining water and other components are contained in the other layer.

In Examples of the present invention, when the phase separation composition comprises a nonionic surfactant, in the step of mixing the phase separation composition, the mixture may be heated during mixing for clearer phase separation. For example, the mixture obtained by mixing the phase separation composition with a primary extract may be stirred while maintaining a temperature of 30 to 50°C, preferably 35 to 45°C.

When a primary extract is mixed with the phase separation composition, the Brevilin A, the Arnicolide D, the Arnicolide C, and the Microhelenin C, which are non-polar natural substances, are extracted with and dissolved in a nonionic surfactant or a lipophilic solubilizer. At this time, when highspeed mixing is additionally performed, the surface area of the surfactant and solubilizer in the mixture is increased, and most of the non-polar natural substances that have not been extracted or dissolved are dissolved, resulting in an instantaneous critical micelle concentration (CMC) state. At that time, when a cloud point is reached as the temperature of the mixed solution increases, since the solubility of nonionic surfactants decreases as the temperature increases, unlike ionic surfactants, the phase separation of the mixed solution becomes more apparent.

The top layer of a phase-separated solution is separated to obtain the Brevilin A, the Arnicolide D, the Arnicolide C, and the Microhelenin C. After the phase-separated solution is left as it is, it is filtered by centrifugation to obtain the *Centipeda minima* extract.

The composition for preventing and treating inflammatory disease and autoimmune diseases and non-alcoholic fatty liver disease comprises the *Centipeda minima* extract obtained, and may further comprise a lipophilic solubilizer, a surfactant, and water.

In addition, with the phase separation layer containing the Brevilin A, the Arnicolide D, the Arnicolide C, and the Microhelenin C extracted according to the extraction method, an emulsification composition in a microemulsion range, which is out of the range of the phase separation composition, may be further mixed to prepare an emulsified product.

Products such as natural substances extracted according to the Examples of the present invention and compositions comprising the same can be prepared without using a liquid-liquid extraction method that uses a toxic solvent, and so steps such as drying can be omitted. In addition, since the solvent is a substance that is harmless to human body, an extract containing natural substances can be directly used as a product without any other steps. In addition, microemulsion or CMC may increase the absorption rate of the product.

In general, the use of a surfactant increases the absorption rate by increasing the solubility of a compound using the CMC of the surfactant. Microemulsion can also increase the absorption rate by not only increasing the solubility but also reducing the size of the material to be absorbed.

The products manufactured in this way can be manufactured as products such as hair tonic, hair conditioner, hair lotion, hair nutrition lotion, hair shampoo, hair rinse, hair treatment, hair cream, hair nutrition cream, hair moisture cream, hair massage cream, hair wax, and hair aerosol, hair pack, hair nutrition pack, hair soap, hair cleansing foam, hair oil, hair drying agent, hair preservative, hair dye, hair wave agent, hair bleach, hair gel, hair glaze, hair dressing, hair lacquer, hair moisturizer, hair mousse, and hairspray; and as formulations for external skin application such as cream, gel, patch, spray, ointment, plaster, lotion, liniment, paste, and cataplasma; and can be appropriately applied in a liquid phase, a solid phase, and a gaseous phase.

Such a product is effective in improving various diseases to which the effects of the Brevilin A, the Arnicolide D, the Arnicolide C and the Microhelenin C can be applied, and may exhibit an effect in preventing and treating autoimmune diseases and inflammation such as, for example, hair loss, alopecia areata, rheumatoid arthritis, psoriasis, atopy, eczema, seborrheic dermatitis, scleroderma, skin immune hypersensitivity, multiple sclerosis, lupus, Behçet's disease, ankylosing spondylitis, Sjogren's syndrome, Crohn's disease, and inflammatory bowel disease.

A preventive and therapeutic agent of inflammatory disease and autoimmune diseases and non-alcoholic fatty liver disease according to another Example of the present invention may comprise the composition for preventing and treating inflammatory disease and autoimmune diseases and non-alcoholic fatty liver disease, and for example, the preventive and therapeutic agent of inflammatory disease and autoimmune diseases and non-alcoholic fatty liver disease may comprise the composition for preventing and treating inflammatory disease and autoimmune diseases and non-alcoholic fatty liver disease, comprising 0.1 to 90% by weight of the *Centipeda minima* extract based on the total weight of the composition.

In the present invention, `composition for preventing and treating' means a composition administered for a specific purpose. According to the purpose of the present invention, the composition for preventing and treating of the present invention may widely vary according to various factors including the activity of the specific compound used, age, body weight, general health conditions, sex, formula, administration time, route of administration, excretion rate, drug combination and specific disease to be prevented or treated. The dosage of the composition for preventing and treating may be appropriately selected by one of ordinary skill in the art depending on the conditions of the patent, body weight, severity of the disease, drug type, and administration route and duration. The composition for preventing and treating according to the present invention may be formulated as pill, sugar-coated pill, capsule, liquid, gel, syrup, slurry, or suspension. The composition for preventing and treating according to the present invention is for preventing and treating inflammatory disease and autoimmune diseases and non-alcoholic fatty liver disease, and a compound involved therein and a pharmaceutically acceptable carrier, excipient or diluent may be included. Carriers, excipients and diluents that may be included in the composition of the present invention include lactose, dextrose, sucrose, sorbitol, mannitol, xylitol, erythritol, maltitol, starch, gum acacia, alginates, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil but are not limited thereto.

Hereinafter, the composition for preventing and treating inflammatory disease and autoimmune diseases and non-alcoholic fatty liver disease of the present invention will be described in more detail through Examples.

The composition for preventing and treating inflammatory disease and autoimmune diseases and non-alcoholic fatty liver disease of the present invention comprises a *Centipeda minima* extract.

### Example

The natural raw material was *Centipeda minima,* and a *Centipeda minima* extract obtained by extracting *Centipeda minima* (Yeongcheon) purchased from the Goesan Medicinal Herb and Organic Agricultural Products Cooperative Association (https://natural-herb.co.kr) in Korea was used.

A primary extract was obtained by immersing 1,000 g of hay of *Centipeda minima* in 10 L of 80% ethanol for 48 hours and performing alcohol cold extraction. Thereafter, the primary extract was concentrated under reduced pressure to remove ethanol.

After mixing 80% by weight of the primary extract concentrated under reduced pressure and 20% by weight of Labrafac as a lipophilic solubilizer, the resulting mixture was stirred for 10 minutes with a circulation type in-line mixer. Then, after allowing to stand as it was for 60 minutes, the phase-separated supernatant was separated.

The *Centipeda minima* extract was named as CMX (CM-2019-001) and stored in the herbarium of Kyungsung University, and the International Nomenclature Cosmetic Ingredient (INCI) raw material number is 33,849.

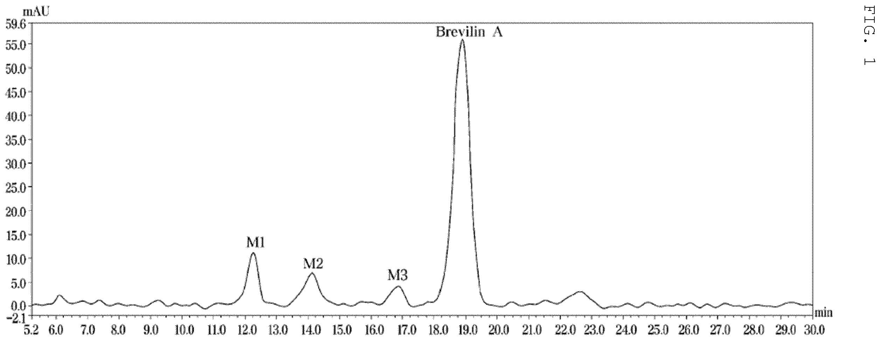

The *Centipeda minima* extract was analyzed by HPLC and mass spectroscopy, and the analytical results showed that active ingredients of Brevilin A, Arnicolide D, Arnicolide C, and Microhelenin C are contained (FIGS. 1 to 6).

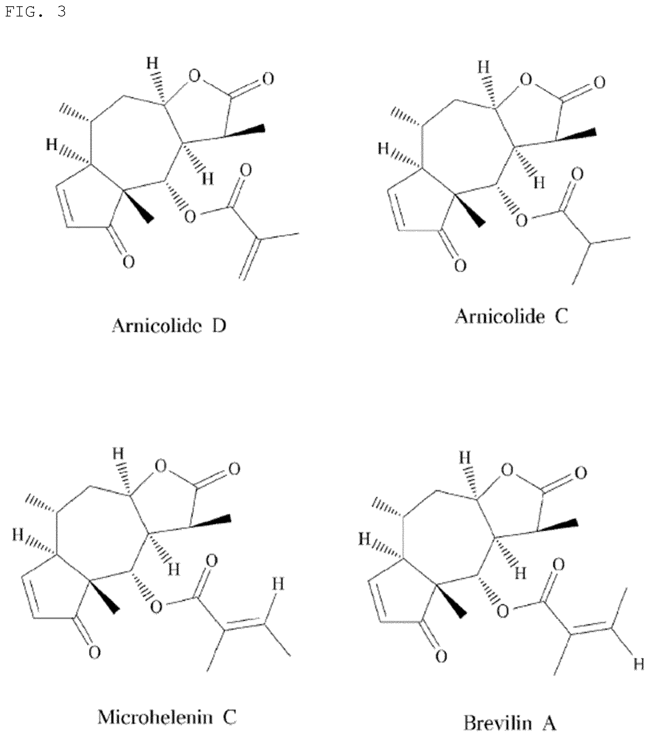

Referring to FIGS. 1 to 6, the results of HPLC analysis of the *Centipeda minima* extract in FIG. 1 show that four main peaks were found. In order to confirm this, as shown in FIG. 3, the results were compared with the graph showing the results of the HPLC analysis of a mixture of Brevilin A, Arnicolide D, Arnicolide C, and Microhelenin C.

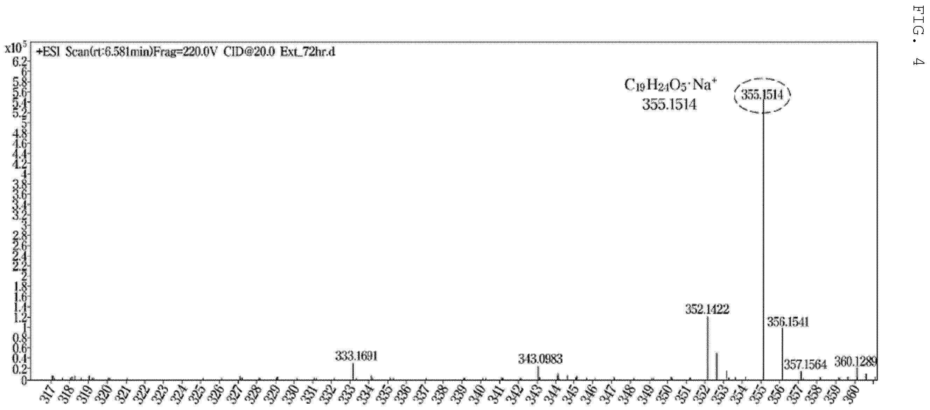

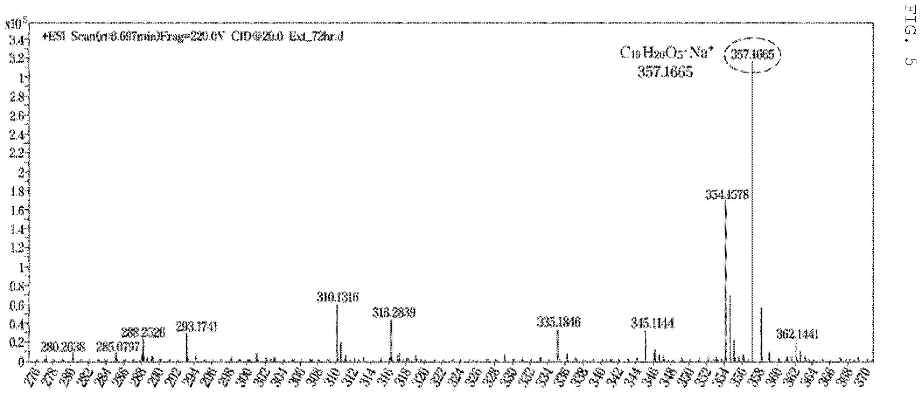

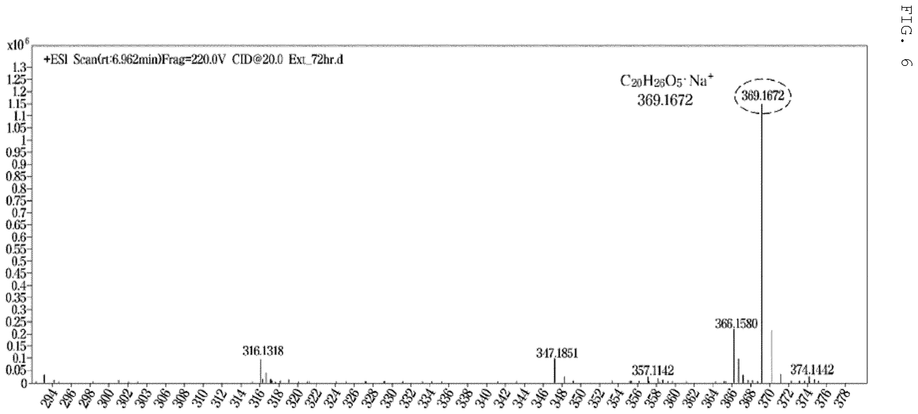

FIGS. 4 to 6 are diagrams showing the UHPLC-Q-TOF mass spectrum to analyze the individual ingredients of the *Centipeda minima* extract according to an Example of the present invention. Arnicolide D, having a molecular formula of C₁₉H₂₄O₅ and a molecular weight of 332.40, corresponds to FIG. 4; Arnicolide C, having a molecular formula of C₁₉H₂₆O₅ and a molecular weight of 334.41, corresponds to FIG. 5; and Microhelenin C, having a molecular formula of C₂₀H₂₆O₅ and a molecular weight of 346.4, corresponds to FIG. 6.

### Experimental Example 1

The present experiment was performed at the Korea Dermatological Research Institute to confirm the hair growth and hair loss effect of the composition for preventing and treating inflammatory disease and autoimmune diseases and non-alcoholic fatty liver disease, comprising the *Centipeda minima* extract.

A sample of the composition for preventing and treating inflammatory disease and autoimmune diseases and non-alcoholic fatty liver disease, comprising the *Centipeda minima* extract, according to the Example, was administered to a test group, and a sample of a composition comprising Labrafac, Labrasol, and D-panthenol was administered to a control group as a placebo control group. The experiment was performed for 24 weeks, and hair growth and hair loss were observed in an interval of 8 weeks.

There were 72 subjects, including 36 subjects in the test group and 36 subjects in the control group who satisfied the criteria described below.
- Male and female subjects diagnosed with androgenetic alopecia between the ages of 37 and 54
- Male research subjects diagnosed as class 2 or 2A or higher by Norwood Hamilton classification and female research subjects diagnosed as class 1 or higher by Ludwig classification.
- Research subjects who would not use special hair products or who would not perform special hair care and manipulation during the research period
- Research subjects who would maintain the same hair style and color during the study period as at the time of the first visit

As an experimental method, the experimental subjects used an appropriate amount of the sample once a day under the supervision of the tester, using it once on the skin after shampooing every evening and sufficiently drying the hair by applying 0.5 mL for each use. Rather than applying the sample to the entire skin in a day, half application was performed by dividing the skin into 2 sections and applying the sample to half of the skin each day over two days.

Use of double-blind method: In sample distribution, the test sample and the control sample were distributed in the same containers so that both the test subjects and the tester could not know the contents of the samples. The sample code used for distinction was sealed so that it could not be exposed, and a classification symbol (A/B) was marked on top of the sample code.

The change of the total hair counts was evaluated with a phototrichogram, and after cutting the hairs on the hair loss area to be evaluated, small dots with a diameter of 1 mm were tattooed or a particular coordinate was recorded. The phototricogram was performed each time around the dot tattoo or the particular coordinate.

The total hair counts (number/cm²) were obtained by observing the number of hairs within a circle of 1 cm².

The results of the test group (total hair counts) are shown in Table 1.

**[Table 1]**

| No. | Week 0 | Week 8 | Week 16 | Week 24 |
|---|---|---|---|---|
| 1 | 44 | 47 | - | - |
| 2 | 35 | 38 | 41 | 42 |
| 3 | 49 | 46 | 49 | 51 |
| 4 | 46 | 47 | 46 | 46 |
| 5 | 39 | 39 | 40 | 43 |
| 6 | 35 | 37 | 37 | 38 |
| 7 | 47 | 48 | 48 | 48 |
| 8 | 44 | 51 | 51 | 53 |
| 9 | 48 | 49 | 48 | 48 |
| 10 | 66 | 68 | 68 | 68 |
| 11 | 42 | 45 | 42 | 46 |
| 12 | 63 | 63 | 63 | 64 |
| 13 | 36 | 38 | 41 | 42 |
| 14 | 41 | 40 | 41 | 42 |
| 15 | 39 | 41 | 40 | 42 |
| 16 | 35 | 36 | 34 | 34 |
| 17 | 52 | 52 | 53 | 54 |
| 18 | 40 | 41 | 38 | 40 |
| 19 | 30 | 30 | 31 | 34 |
| 20 | 64 | 64 | 66 | 70 |
| 21 | 52 | 53 | 55 | 57 |
| 22 | 50 | 52 | 53 | 54 |
| 23 | 53 | 51 | 54 | 54 |
| 24 | 48 | 48 | 48 | 49 |
| 25 | 33 | 37 | 38 | 38 |
| 26 | 33 | 36 | 35 | 36 |
| 27 | 37 | 36 | 41 | 43 |
| 28 | 44 | 45 | 46 | 48 |
| 29 | 46 | 50 | 50 | 52 |
| 30 | 40 | 40 | 40 | 41 |
| 31 | 33 | 33 | 35 | 37 |
| 32 | 37 | - | - | - |
| 33 | 31 | 34 | 34 | 36 |
| 34 | 36 | 37 | 39 | 41 |
| 35 | 39 | 40 | 41 | 42 |
| 36 | 51 | 51 | 54 | 54 |

The results of the control group (total hair counts) are shown in Table 2.

**[Table 2]**

| No. | Week 0 | Week 8 | Week 16 | Week 24 |
|---|---|---|---|---|
| 1 | - | - | - | - |
| 2 | 37 | 33 | 38 | - |
| 3 | 35 | 34 | 33 | 33 |
| 4 | 50 | 50 | 52 | 53 |
| 5 | 42 | 42 | 42 | 41 |
| 6 | 42 | 44 | 34 | 39 |
| 7 | 38 | 38 | 38 | 39 |
| 8 | 58 | 59 | 59 | 62 |
| 9 | 42 | 44 | 42 | 41 |
| 10 | 46 | 46 | 46 | 46 |
| 11 | 48 | 47 | 48 | 50 |
| 12 | 49 | 49 | 50 | 50 |
| 13 | 49 | 51 | 50 | 52 |
| 14 | 38 | 38 | 37 | 35 |
| 15 | 48 | 49 | 46 | 49 |
| 16 | 31 | 32 | 31 | 30 |
| 17 | 48 | 45 | 43 | 44 |
| 18 | 42 | 45 | 45 | 48 |
| 19 | 51 | 51 | 51 | 54 |
| 20 | 37 | 33 | 33 | 37 |
| 21 | 51 | 50 | 50 | 51 |
| 22 | 55 | 54 | 54 | 53 |
| 23 | 46 | 46 | 47 | 47 |
| 24 | - | - | - | - |
| 25 | 36 | 36 | 37 | 35 |
| 26 | 26 | 25 | 27 | 27 |
| 27 | 27 | 26 | 33 | 31 |
| 28 | 39 | 39 | 39 | 39 |
| 29 | 52 | 52 | 52 | 52 |
| 30 | 39 | 38 | 40 | 37 |
| 31 | 35 | 35 | 36 | 35 |
| 32 | 35 | 37 | 38 | 36 |
| 33 | 48 | 47 | 45 | 46 |
| 34 | 32 | 33 | 27 | 32 |
| 35 | 45 | 41 | 45 | 42 |
| 36 | 41 | 44 | - | - |

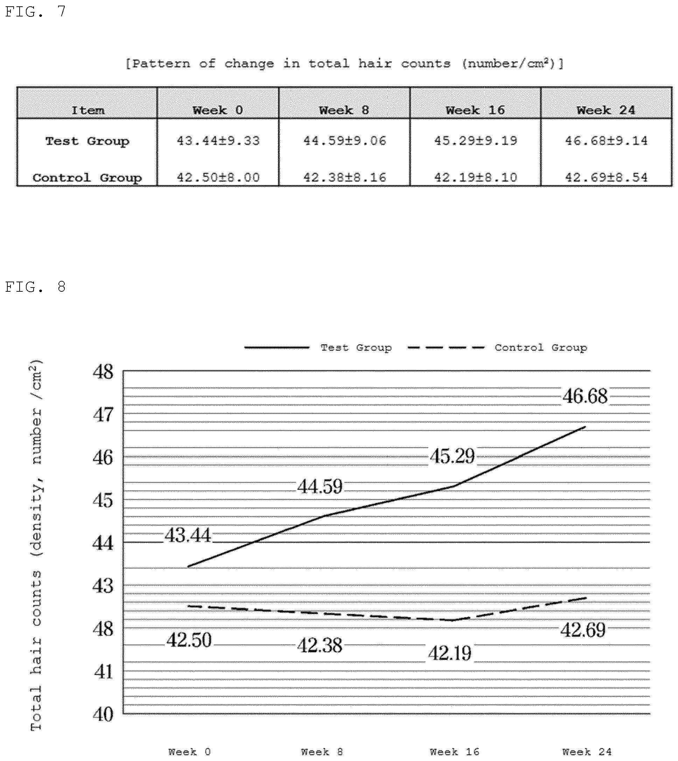

Referring to Tables 1 and 2 above and FIGS. 7 to 9, in the case of the test group, it was confirmed that hair growth or hair loss was improved as the total hair counts were increased from Week 8, and in the case of the control group, it was confirmed that there was little change or rather the total hair counts were decreased in Week 8 and Week 16.

For a secondary efficacy evaluation, the degree of hair improvement was visually evaluated by the tester by photography. Photos of the human body were always taken at a predetermined position under the same conditions by using a high-resolution digital camera (DSLR) and compared before and after the application of the test sample.
- Shooting of photos of human body: Photos of two areas at an angle of 45 degree (frontal line) and 90 degree (top of the head) were taken under the same conditions and at the same composition and position at each evaluation time point.
- Photo evaluation: For visual evaluation, two researchers performed the visual evaluation on a 7-level scale and the average of the two visual evaluation results was used as an efficacy endpoint.

**[Table 3]**

| Much improved | Improved | Slightly improved | No change 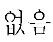 | Slightly worsened | Worsened | Much worsened |
|---|---|---|---|---|---|---|
| +3 | +2 | +1 | 0 | -1 | -2 | -3 |

The degree of improvement of the hair of the test group was evaluated, and the average of the evaluation results is shown in Table 4 below.

**[Table 4]**

| No. | Week 8 | Week 16 | Week 24 |
|---|---|---|---|
| 1 | -1.5 | - | - |
| 2 | 0 | 1 | 1 |
| 3 | 0 | 0 | 0 |
| 4 | 0 | 0 | 0.5 |
| 5 | 0 | 0 | 0.5 |
| 6 | -1 | 1 | 1 |
| 7 | -1 | 1 | 1 |
| 8 | 0 | 0.5 | 1.5 |
| 9 | -0.5 | 0.5 | 1 |
| 10 | 0 | 0 | 0.5 |
| 11 | 1 | 1 | 1 |
| 12 | -0.5 | 1 | 1 |
| 13 | 0 | 0 | 0.5 |
| 14 | 0 | 0.5 | 1 |
| 15 | 0 | 1 | 2 |
| 16 | 0 | 0 | 0 |
| 17 | 0 | 1 | 1 |
| 18 | 0 | 1 | 1 |
| 19 | -0.5 | 0.5 | 1 |
| 20 | 0 | 0 | 0 |
| 21 | -0.5 | 0.5 | 1.5 |
| 22 | -1.5 | 0.5 | 0 |
| 23 | 0 | 1 | 1 |
| 24 | 0 | 1 | 1 |
| 25 | 1 | 2 | 2 |
| 26 | -1 | 1 | 0.5 |
| 27 | 0 | 0.5 | 0.5 |
| 28 | -0.5 | 0.5 | 0.5 |
| 29 | 0.5 | 1 | 1 |
| 30 | 1 | 2 | 2 |
| 31 | -1 | 0 | -0.5 |
| 32 | - | - | - |
| 33 | 0 | 0 | 0 |
| 34 | -0.5 | 0 | 0.5 |
| 35 | 0.5 | 1 | 1 |
| 36 | 0 | 1 | 1 |

The degree of improvement of the hair of the control group was evaluated, and the average of the evaluation results is shown in Table 5 below.

**[Table 5]**

| No. | Week 8 | Week 16 | Week 24 |
|---|---|---|---|
| 1 | - | - | - |
| 2 | 0 | 0 | - |
| 3 | 0 | 0 | 0 |
| 4 | -0.5 | 0.5 | 0.5 |
| 5 | -0.5 | 0 | 0 |
| 6 | -0.5 | 0 | 0 |
| 7 | 0 | -1 | -1 |
| 8 | 0 | 0 | 1 |
| 9 | 0 | 0 | 0 |
| 10 | 0 | 0 | -1 |
| 11 | 0 | 1 | 1 |
| 12 | 0 | 0 | 1 |
| 13 | 0 | 0 | 0 |
| 14 | 0 | 0 | 0 |
| 15 | -1 | -1 | 0 |
| 16 | 0 | 0 | 0.5 |
| 17 | 0 | 0 | 0 |
| 18 | -0.5 | 0 | 0 |
| 19 | 0 | 0 | 0 |
| 20 | 0 | -1 | -1 |
| 21 | 0 | 0.5 | 0 |
| 22 | 0 | 0 | 0 |
| 23 | 0 | 0 | 0 |
| 24 | - | - | - |
| 25 | 0 | -1 | -1 |
| 26 | 0 | 1 | 1 |
| 27 | 0 | 0 | 0 |
| 28 | 0 | 0 | 0 |
| 29 | 0 | 1 | 1 |
| 30 | 0 | 1 | 1 |
| 31 | 0 | 0 | 0 |
| 32 | 0 | -0.5 | 0 |
| 33 | 0 | 0 | 0 |
| 34 | -1 | 0 | 0 |
| 35 | 0 | -1 | -1 |
| 36 | -0.5 | - | - |

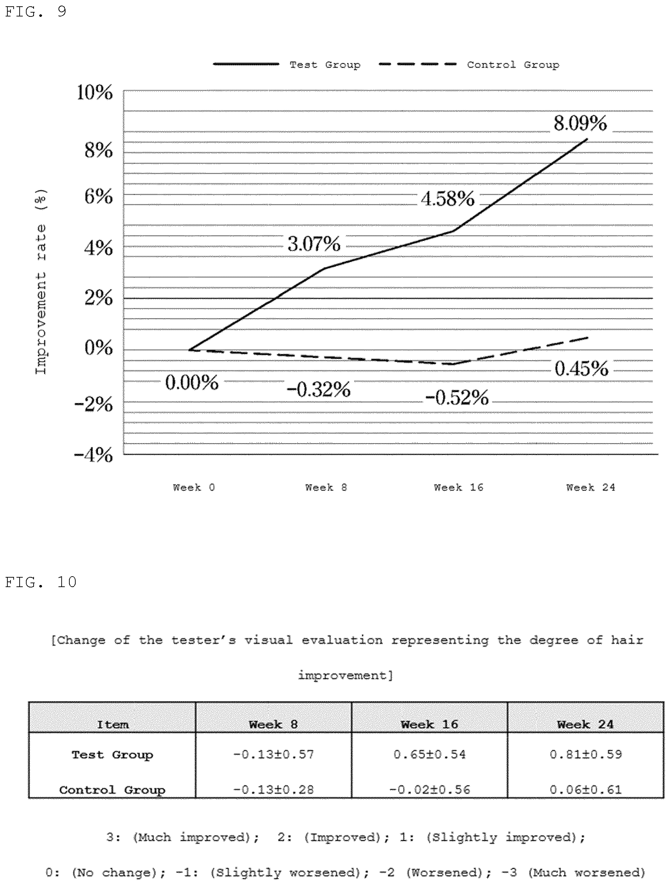

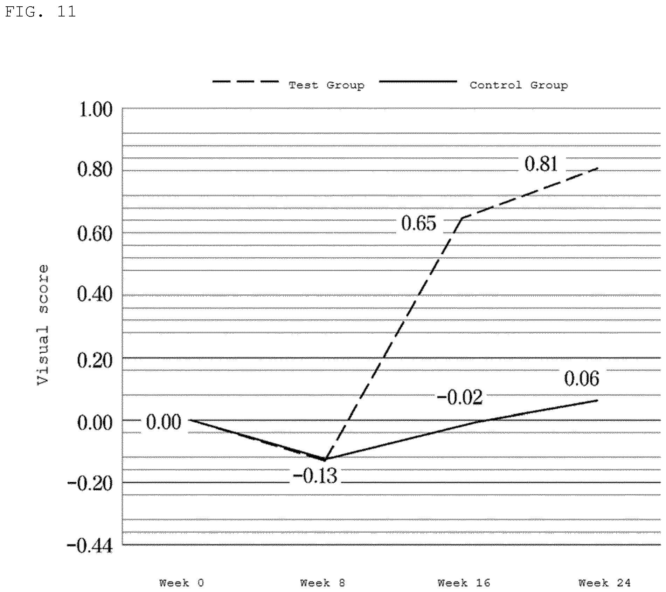

Referring to Tables 3 to 5 above and FIGS. 10 and 11, the test group and the control group were at almost similar levels until Week 8, However, it was confirmed that the visual evaluation score in the test group increased remarkably from Week 16 onwards and that there was almost no change or only a slight increase in the control group.

### Experimental Example 2: Anti-inflammatory efficacy 1

To confirm the anti-inflammatory effect of the *Centipeda minima* extract, an experiment was performed with inflammation-related factors Ccl2, IL-1b, TNF-a, and IL-10.

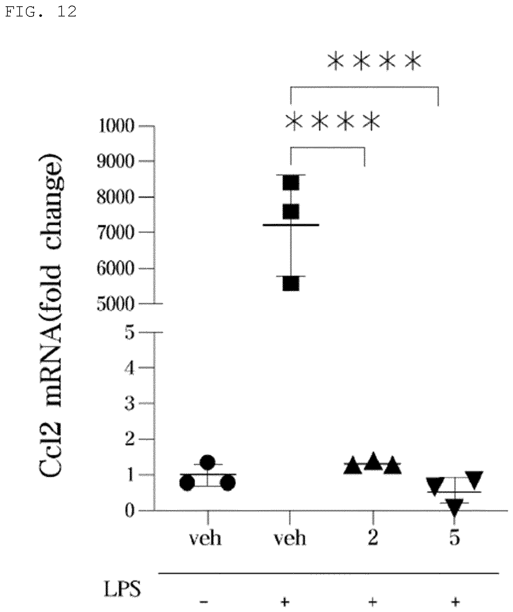

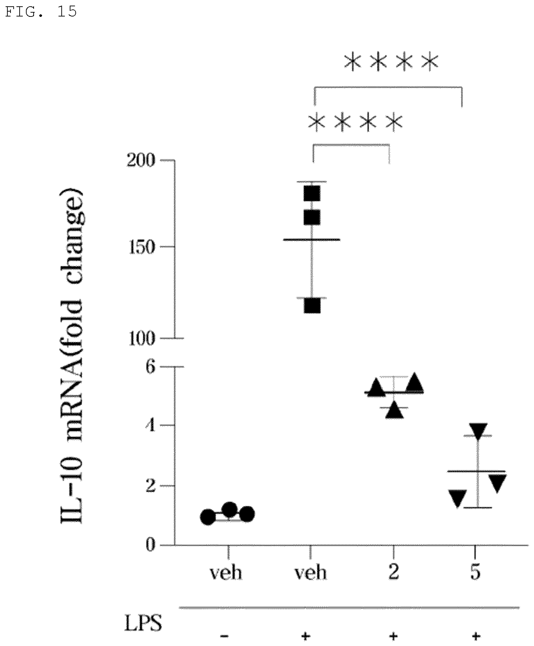

FIGS. 12 to 15 are diagrams confirming the contents of inflammation-related factors Ccl2, IL-1b, TNF-a, and IL-10 to confirm the anti-inflammatory effect of the composition for preventing and treating anti-inflammatory and autoimmune diseases and non-alcoholic fatty liver disease according to an Example of the present invention.

In FIGS. 12 to 15, as Comparative Examples, 'Veh-' is a negative control group, 'Veh+ is' a positive control group, and '2' corresponds to the application of 6 ug of Brevilin A alone. As an Example, '5' corresponds to the *Centipeda minima* extract according to an Example of the present invention, wherein the composition comprises Brevilin A 2.22 µg, Arnicolide C 0.21 µg, Arnicolide D 0.6 µg, and Microhelenin C 0.14 µg, and the total amount of Brevilin A, which is the active substance, and Arnicolide C, Arnicolide D, and Microhelenin C, which are marker substances, was 3.17 µg.

Specifically, the anti-inflammatory effect 1 was evaluated by using RAW 264.7 macrophages, and LPS (250 ng/ml) was treated in the RAW 264.7 cells to stimulate the production of each of Ccl2, IL-1b, TNF-a, and IL-10. Thereafter, the cells were treated with 6 µg of Brevilin A alone (2); and with the *Centipeda minima* extract according to an Example of the present invention, comprising Brevilin A 2.22 µg, Arnicolide C 0.21 µg, Arnicolide D 0.6 µg, and Microhelenin C 0.14 µg (5). After culturing for 6 hours under the same conditions, the cells were removed, and the amounts of Ccl2, IL-1b, TNF-a, and IL-10 were quantified by ELISA. Accordingly, changes in the Ccl2, IL-1b, TNF-a and IL-10 protein expression by the composition for preventing and treating inflammatory disease and autoimmune diseases and non-alcoholic fatty liver disease were analyzed in the RAW cells.

Referring to FIG. 12, with regard to the change in the Ccl2 protein expression, the effect was higher in the case where the *Centipeda minima* extract (5) of an Example of the present invention was used than in the case where Brevilin A was used alone (2).

Referring to FIG. 12, with regard to the change in the IL-1b protein expression, the effect in the case where the *Centipeda minima* extract (5) of an Example of the present invention was used was similar or equivalent to the case where Brevilin A was used alone (2). Considering that the effect of a smaller dosage was similar or equivalent to the case where Brevilin A was used alone (2) in the Comparative Example, it was found that the *Centipeda minima* extract (5) has a better anti-inflammatory effect.

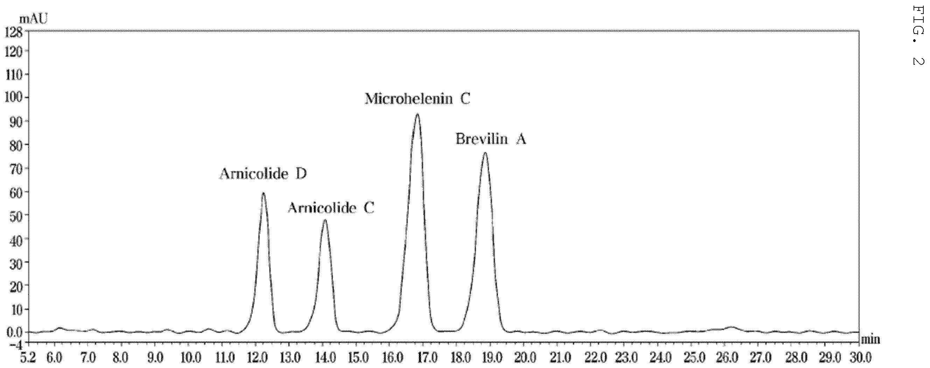

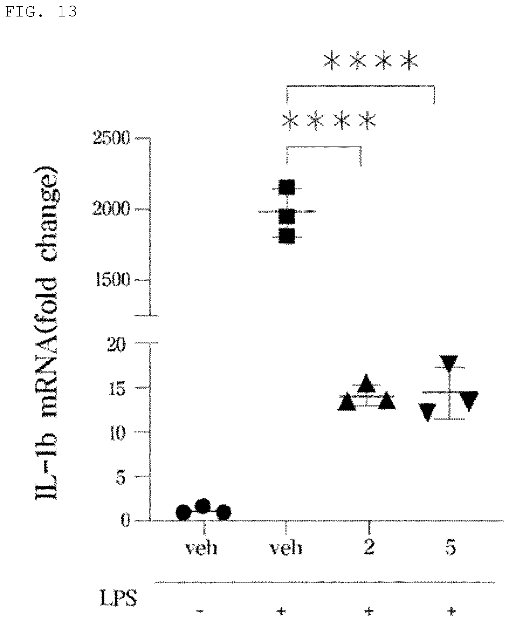

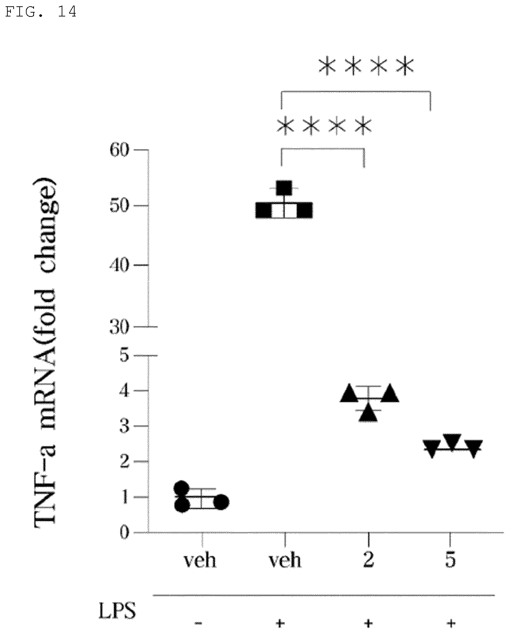

Referring to FIGS. 13 and 14, with regard to the change of the TNF-a and IL-10 protein expression, as in the Ccl2 protein expression change shown in FIG. 2, it was confirmed that the *Centipeda minima* extract (5) of an Example of the present invention has a better anti-inflammatory effect than in the case where Brevilin A was used alone (2).

### Experimental Example 3: Anti-inflammatory efficacy 2

To confirm the anti-inflammatory effect of the composition for preventing and treating inflammatory disease and autoimmune diseases and non-alcoholic fatty liver disease, comprising the *Centipeda minima* extract, according to the composition ratio, the composition ratios (% by weight) of the active ingredients were adjusted as shown in Table 6 below.

**[Table 6]**

| | Brevilin A | Arnicolide D | Arnicolide C | Microhelenin C |
|---|---|---|---|---|
| Example 1 | 70 | 7 | 19 | 4 |
| Example 2 | 80 | 20 | - | - |
| Example 3 | 80 | - | 20 | - |
| Example 4 | 85 | - | - | 15 |
| Example 5 | 70 | 10 | 20 | - |
| Example 6 | 70 | - | 20 | 10 |
| Comparative Example 1 | 28 | 28 | 29 | 15 |
| Comparative | 92 | 3 | 3 | 2 |
| Example 2 | | | | |
| Comparative Example 3 | 77 | 10 | 3 | 10 |
| Comparative Example 4 | 55 | 35 | 5 | 5 |
| Comparative Example 5 | 80 | 5 | 13 | 2 |
| Comparative Example 6 | 100 | - | - | - |

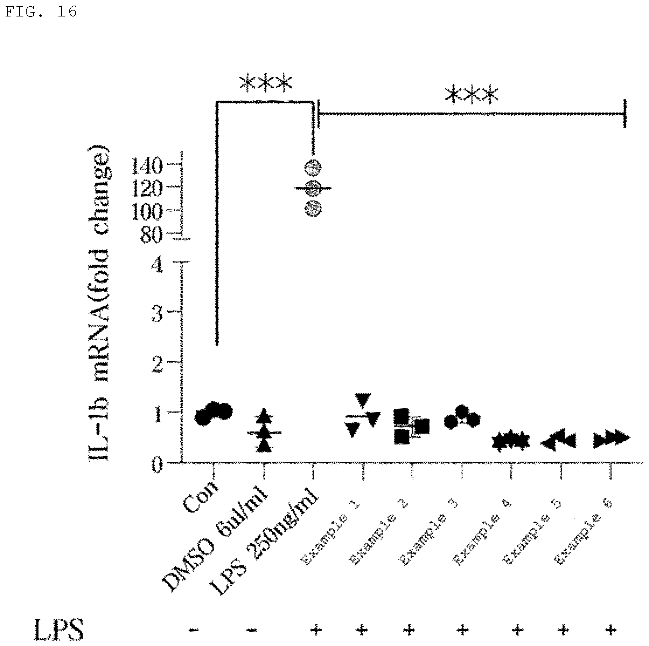

FIGS. 16 to 21 are diagrams confirming the contents of inflammation-related factors IL-1b, IL-6, and TNF-a to confirm the anti-inflammatory effect of the composition for preventing and treating inflammatory disease and autoimmune diseases and non-alcoholic fatty liver disease according to an Example of the present invention.

Specifically, the anti-inflammatory effect 2 was evaluated by using RAW 264.7 macrophages, and LPS (250 ng/ml) was treated in the RAW 264.7 cells to stimulate the production of each of IL-1b, IL-6, and TNF-a. Each of Examples 1 to 6 and Comparative Examples 1 to 6 was treated at 6 ug/ml and cultured under the same conditions for 6 hours, and then the cells were removed and the amounts of IL-1b, IL-6, and TNF-a were quantified by ELISA. Accordingly, changes in the IL-1b, IL-6, and TNF-a protein expression by the composition for preventing and treating inflammatory disease and autoimmune diseases and non-alcoholic fatty liver disease were analyzed in the RAW cells.

*P<0.05 is statistically significant, but in the case of cytotoxicity tests, significance is generally recognized from **P<0.01. Therefore, in the present invention, **P<0.01, ***P<0.001, and ****P< 0.0001 were considered as significant compared to the control group.

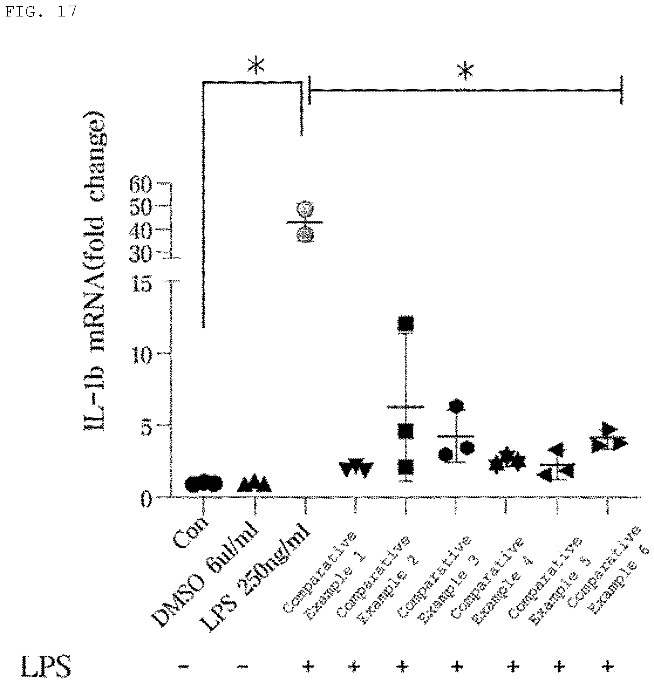

Referring to FIGS. 16 and 17, changes in the IL-1b protein expression changes were analyzed, and when the compositions of Examples 1 to 6 were treated, the IL-1b protein expression was significantly decreased compared to Comparative Examples 1 to 6.

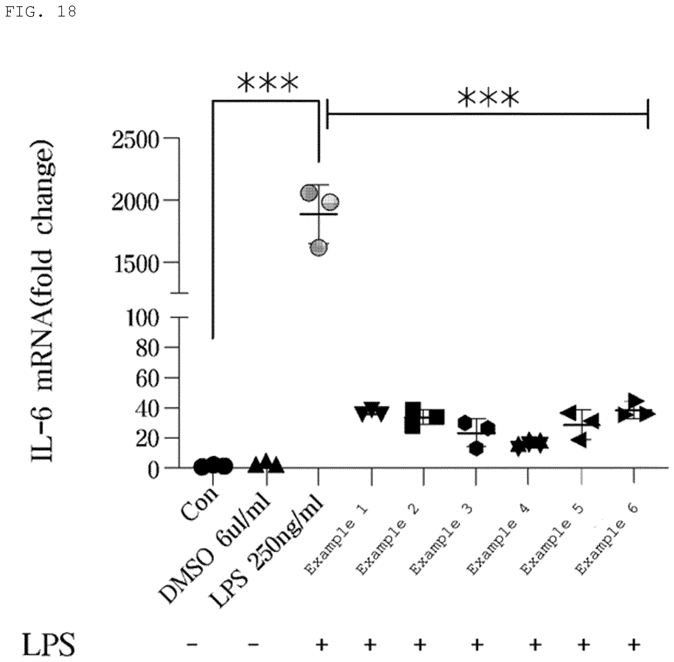

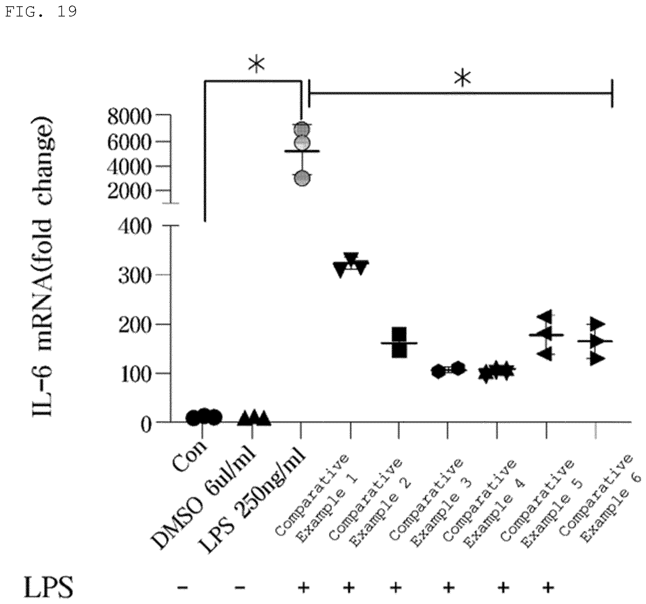

Referring to FIGS. 18 and 19, changes in the IL-6 protein expression changes were analyzed, and when the compositions of Examples 1 to 6 were treated, the IL-6 protein expression was significantly decreased compared to Comparative Examples 1 to 6.

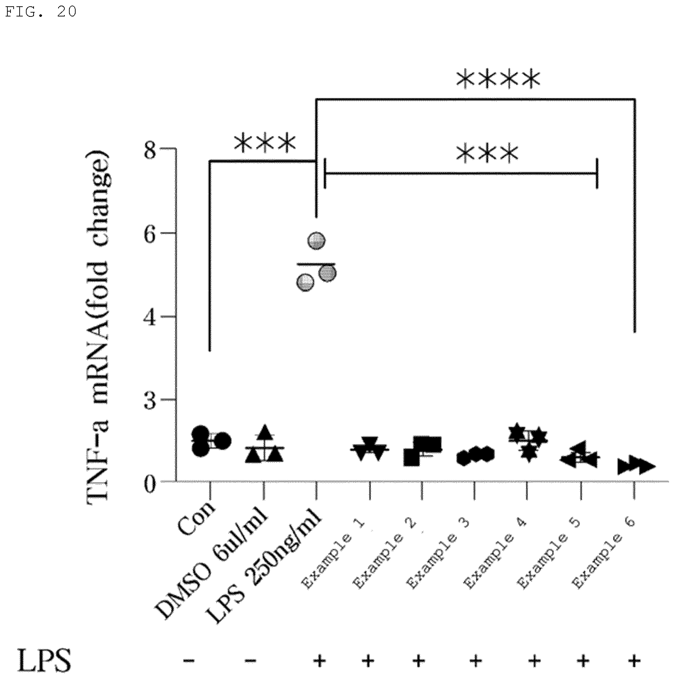

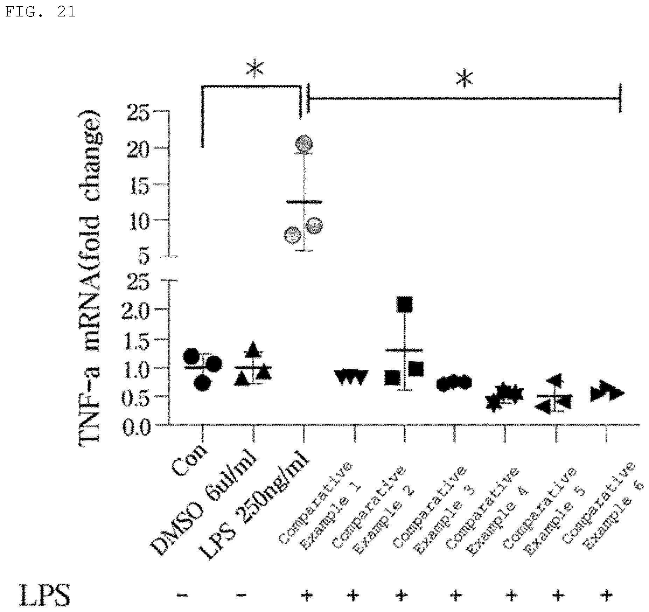

Referring to FIGS. 20 and 21, changes in the TNF-a protein expression changes were analyzed, and when the compositions of Examples 1 to 6 were treated, the TNF-a protein expression was significantly decreased compared to Comparative Examples 1 to 6.

In terms of significance, the Comparative Examples showed a significance level of *P<0.05, but the Examples showed a significance level of ***P<0.001 or more. Therefore, it was confirmed that the Examples showed much better anti-inflammatory efficacy than the Comparative examples.

### Experimental Example 4: Non-alcoholic steatohepatitis inhibitory effect 1 - Adipocyte differentiation inhibitory effect

To confirm the effect of the *Centipeda minima* extract for inhibiting non-alcoholic steatohepatitis, an experiment was performed on preadipocytes according to an inflammatory disease treatment agent.

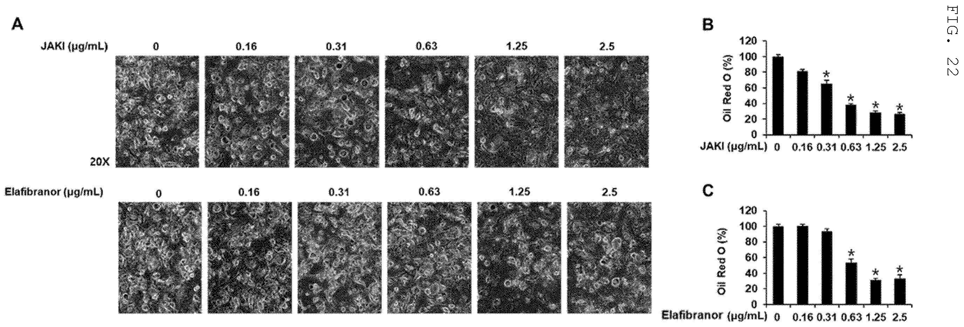

FIG. 22 is a diagram for confirming the effect of a preventive and therapeutic agent of inflammatory disease and autoimmune diseases and non-alcoholic fatty liver disease according to an Example of the present invention and a control group for inhibiting adipocyte differentiation. JAKI is a composition for preventing and treating inflammatory disease and autoimmune diseases and non-alcoholic fatty liver disease according to an Example of the present invention, and elafibranor (Genfit) is a new drug candidate for preventing and treating non-alcoholic fatty liver disease.

Specifically, the evaluation was performed by using 3T3-L1 cells, which are preadipocytes, and after the 3T3-L1 cells were treated with each of JAKI and elafibranor at concentrations of 0 µg/ml, 0.16 µg/ml, 0.31 µg/ml, 0.63 µg/ml, and 1.25 µg/ml and then cultured for 6 hours under the same conditions, the changes of the 3T3-L1 cells were analyzed.

Referring to FIG. 22, it was confirmed that the therapeutic agent JAKI according to an Example of the present invention exhibited a higher effect on the inhibition of the differentiation into adipocytes than the treatment with elafibranor.

Specifically, referring to (B) and (C) of FIG. 2, elafibranor was effective in inhibiting adipocyte differentiation as the concentration increased from the treatment at a concentration of 0.63 ug/ml, whereas JAKI exhibited an excellent effect in the adipocyte differentiation inhibition from the treatment at a concentration of 0.31 ug/ml. Referring to (A) of FIG. 22, it was visually confirmed that JAKI had a higher effect on the adipocyte differentiation inhibition than elafibranor.

### Experimental Example 5: Non-alcoholic steatohepatitis inhibitory effect 2 - Presence of preadipocyte toxicity

To confirm the presence of toxicity of the *Centipeda minima* extract to hepatocytes, an experiment was performed with preadipocytes.

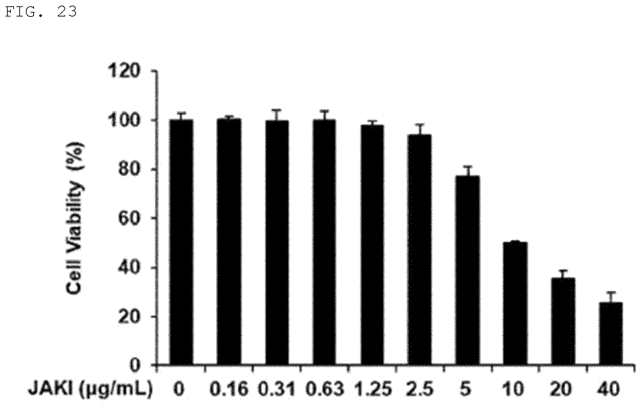

FIG. 23 is a diagram for confirming the presence of cytotoxicity to hepatocytes of a preventive and therapeutic agent of inflammatory disease and autoimmune diseases and non-alcoholic fatty liver disease according to an Example of the present invention and a control group to hepatocytes.

Specifically, the evaluation was performed by using 3T3-L1 cells, which are preadipocytes, and after the 3T3-L1 cells were treated with each of JAKI and elafibranor at concentrations of 0 µg/ml, 0.16 µg/ml, 0.31 µg/ml, 0.63 µg/ml, 1.25 µg/ml, 2.5 µg/ml, 5 µg/ml, 10 µg/ml, 20 µg/ml, and 40 µg/ml and then cultured for 6 hours under the same conditions, the changes of the 3T3-L1 cells were analyzed.

Referring to FIG. 23, the viability was 90% or higher up to a concentration of about 2.5 ug/ml of JAKI according to an Example of the present invention, indicating that there was not damage to the adipocytes. However, the cell viability was lowered to 50% or less at a concentration of 10 µg/ml or higher.

### Experimental Example 6: Non-alcoholic steatohepatitis inhibitory effect 3 - Anti-inflammatory effect in preadipocytes

To confirm the non-alcoholic steatohepatitis inhibitory effect of the *Centipeda minima* extract, an experiment was performed with TNF-α induced IL-6 production in preadipocytes according to an inflammatory disease treatment agent.

IL-6 directly acts on immune responses in a body to induce inflammatory diseases or acute phase reactants in hepatocytes, causing inflammation of hepatocytes or fibrosis of the liver. Therefore, when the IL-6 production is inhibited, non-alcoholic steatohepatitis may be inhibited.

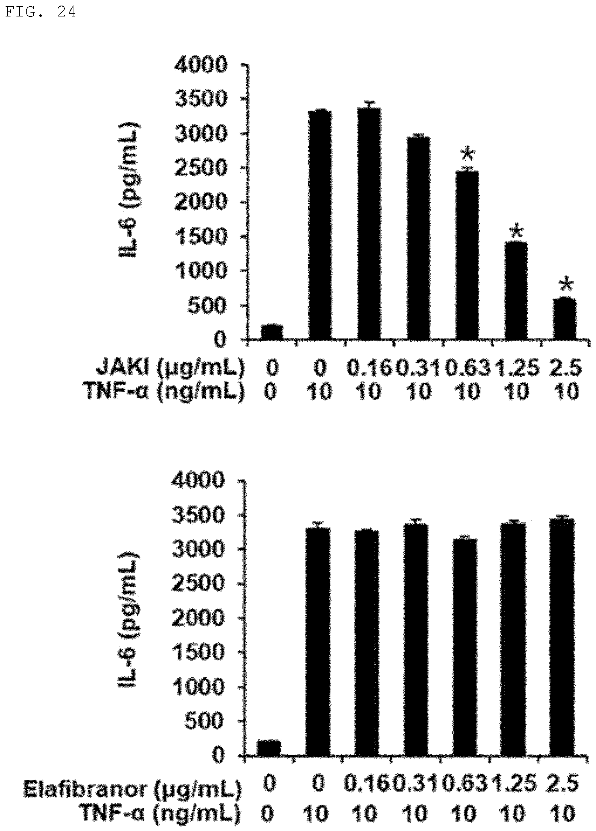

FIG. 24 is a diagram for confirming the effect of a preventive and therapeutic agent of inflammatory disease and autoimmune diseases and non-alcoholic fatty liver disease according to an Example of the present invention and a control group on TNF-α induced IL-6 production. JAKI is a composition for preventing and treating inflammatory disease and autoimmune diseases and non-alcoholic fatty liver disease according to an Example of the present invention, and elafibranor (Genfit) is a new drug candidate for preventing and treating non-alcoholic fatty liver disease.

Specifically, the evaluation was performed by using 3T3-L1 cells, which are preadipocytes, and after the 3T3-L1 cells were treated with each of JAKI and elafibranor at concentrations of 0 µg/ml, 0.16 µg/ml, 0.31 µg/ml, 0.63 µg/ml, 1.25 µg/ml, and 2.5 µg/ml and then cultured for 6 hours under the same conditions, the changes of the IL-6 production were analyzed.

Referring to FIG. 24, the IL-6 production began to be decreased when 0.63 ug/ml of the therapeutic agent JAKI according to an Example of the present invention was treated, and was decreased by more than 50% compared to the uncreated group when JAKI was treated at 1.25 ug/ml. When JAKI was treated at 2.5 µg/ml, the IL-6 production was significantly decreased to about 500 pg/ml or less.

In addition, it was confirmed that there was no change in the IL-6 production when elafibranor was treated.

Accordingly, considering that when JAKI according to an Example of the present invention was treated at a concentration of 0.63 µg/ml or higher, the IL-6 production was decreased, and when JAKI was treated at 1.25 µg/ml, the IL-6 production was significantly decreased by more than 50% of the untreated group, it was found that JAKI showed much better anti-inflammatory efficacy compared to elafibranor.

### Experimental Example 7: Non-alcoholic steatohepatitis inhibitory effect 4 - Insulin resistance inhibitory effect in preadipocytes

To confirm the non-alcoholic steatohepatitis inhibitory effect of the *Centipeda minima* extract, an experiment was performed with TNF-α induced MCP-1 production in preadipocytes according to an inflammatory disease treatment agent.

MCP-1 affects the extracellular signal-regulated kinase (ERK) pathway and induces insulin resistance, causing hepatocyte inflammation or hepatic fibrosis. Therefore, when the MCP-1 production is inhibited, non-alcoholic steatohepatitis may be inhibited.

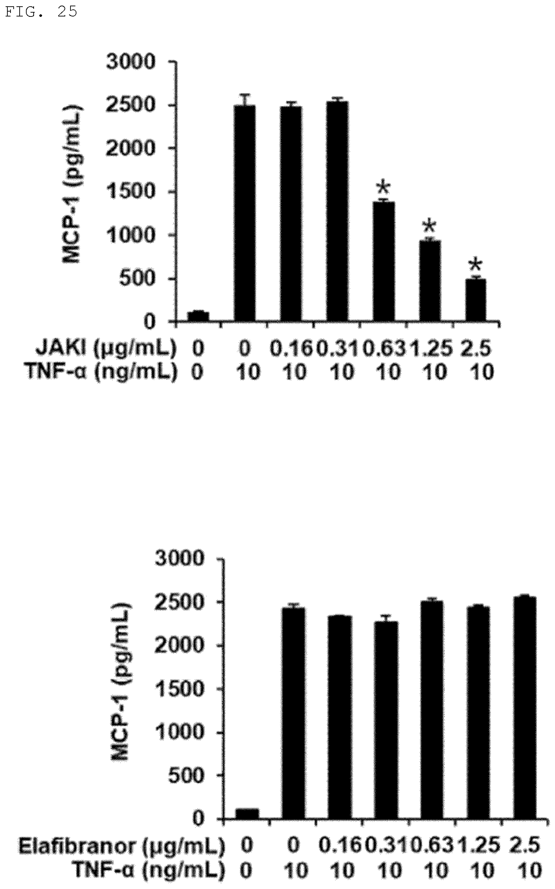

FIG. 25 is a diagram for confirming the effect of a preventive and therapeutic agent of inflammatory disease and autoimmune diseases and non-alcoholic fatty liver disease according to an Example of the present invention and a control group on TNF-α induced MCP-1 production. JAKI is a composition for preventing and treating inflammatory disease and autoimmune diseases and non-alcoholic fatty liver disease according to an Example of the present invention, and elafibranor (Genfit) is a new drug candidate for preventing and treating non-alcoholic fatty liver disease.

Specifically, the evaluation was performed by using 3T3-L1 cells, which are preadipocytes, and after the 3T3-L1 cells were treated with each of JAKI and elafibranor at concentrations of 0 µg/ml, 0.16 µg/ml, 0.31 µg/ml, 0.63 µg/ml, 1.25 µg/ml, and 2.5 µg/ml and then cultured for 6 hours under the same conditions, the changes of the MCP-1 production were analyzed.

Referring to FIG. 25, the MCP-1 production began to be decreased when 0.63 ug/ml of the therapeutic agent JAKI according to an Example of the present invention was treated, and was decreased by more than 50% compared to the uncreated group when JAKI was treated at 1.25 ug/ml. When JAKI was treated at 2.5 ug/ml, the MCP-1 production was significantly decreased to about 500 pg/ml or less.

Referring to FIG. 25, it was confirmed that there was no change in the MCP-1 production when elafibranor was treated.

Accordingly, considering that when JAKI according to an Example of the present invention was treated at a concentration of 0.63 µg/ml or higher, the MCP-1 production was significantly decreased, it was found that JAKI showed much better insulin resistance inhibitory efficacy compared to elafibranor.

### Experimental Example 8: Non-alcoholic steatohepatitis inhibitory effect 5 - Cell viability of hepatic stellate cells

To confirm the non-alcoholic steatohepatitis inhibitory effect of the *Centipeda minima* extract, an experiment was performed with liver fibrosis inhibition-related protein expression according to an inflammatory disease treatment agent.

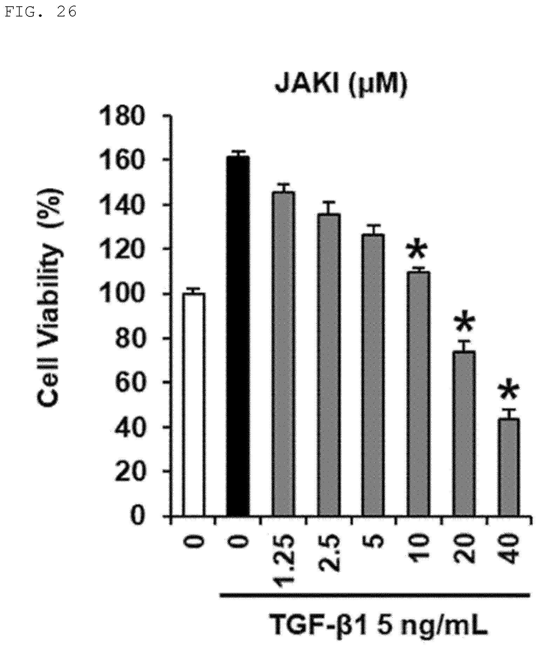

FIG. 26 is a diagram for confirming the cell viability of hepatic stellate cells by a preventive and therapeutic agent of inflammatory disease and autoimmune diseases and non-alcoholic fatty liver disease according to an Example of the present invention. JAKI is a preventive and therapeutic agent of inflammatory disease and autoimmune diseases and non-alcoholic fatty liver disease according to an Example of the present invention.

Specifically, the evaluation was performed by using LX-2 cells, which are hepatic stellate cells, and after the LX-2 cells were activated by treating the LX-2 cells with TGF-β1 at a concentration of 5 ng/ml, JAKI was treated at concentrations of 0 µM, 1.25 µM, 2.5 µM, 5 µM, 10 µM, 20 µM, and 40 µM and the cells were cultured for 6 hours under the same conditions. Then, the changes of the LX-2 cells were analyzed.

Referring to FIG. 26, it was confirmed that when the therapeutic agent JAKI according to an Example of the present invention was treated at 20 µM, the viability of LX-2 cells was decreased, and when JAKI was treated at 40 µM, the viability of LX-2 cells was significantly decreased to 50% or less.

Accordingly, it was found that the therapeutic agent JAKI according to an Example of the present invention is effective in inhibiting the viability of LX-2 cells that induce hepatic fibrosis.

### Experimental Example 9: Non-alcoholic steatohepatitis inhibitory effect 6 - liver fibrosis-related protein expression

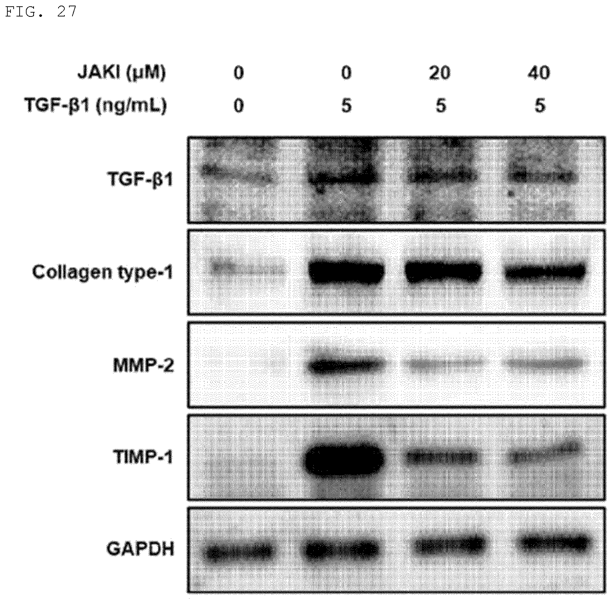

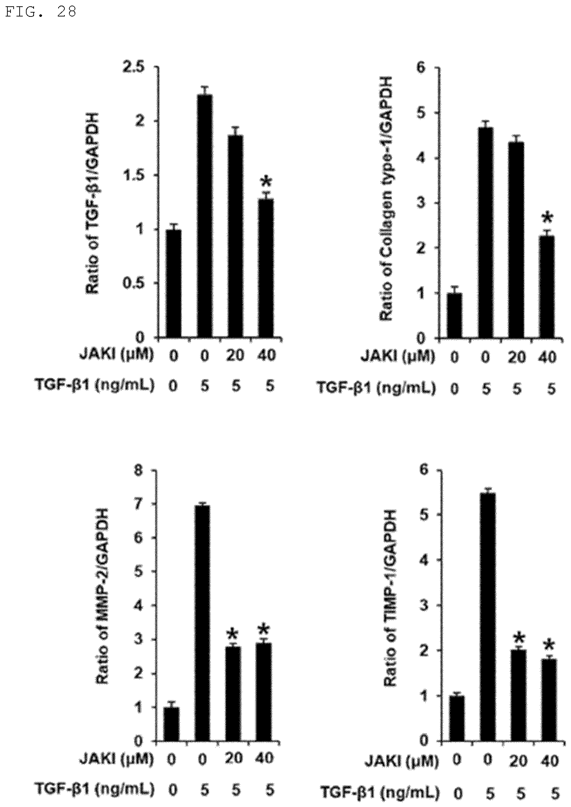

FIGS. 27 and 28 are diagrams for confirming changes in liver fibrosis-related protein expression by a preventive and therapeutic agent of inflammatory disease and autoimmune diseases and non-alcoholic fatty liver disease according to an Example of the present invention.

Specifically, the evaluation was performed by using LX-2 cells, which are hepatic stellate cells. LX-2 cells treated with TGF-β1 at a concentration of 5 ng/ml were treated with JAKI at concentrations of 0 µM, 20 µM, and 40 µM, and then cultured under the same conditions for 24 hours. Thereafter, the protein expression levels of TGF-β1, collagen type-1, MMP-2, TIMP1, and GAPDH, and the protein expression ratios of TGF-β1, collagen type-1, MMP-2, and TIMP1 compared to GAPDH were analyzed.

Referring to FIG. 27, it was confirmed that as the concentration of JAKI was increased, the thickness or color of the bands representing the protein amount of TGF-β1, collagen type-1, and MMP-2 became thinner or lighter, while the thickness or color of the band representing the protein amount of GAPDH remained constant.

Referring to FIG. 28, it was confirmed that when JAKI was treated at 40 µM, the expression ratio of TGF-β1 and collagen type-1 protein compared to GAPDH was significantly decreased, and when JAKI was treated at 20 µM, the expression ratio of MMP-2 and TIMP1 proteins compared to GAPDH was decreased by about 50% or more compared to the untreated group.

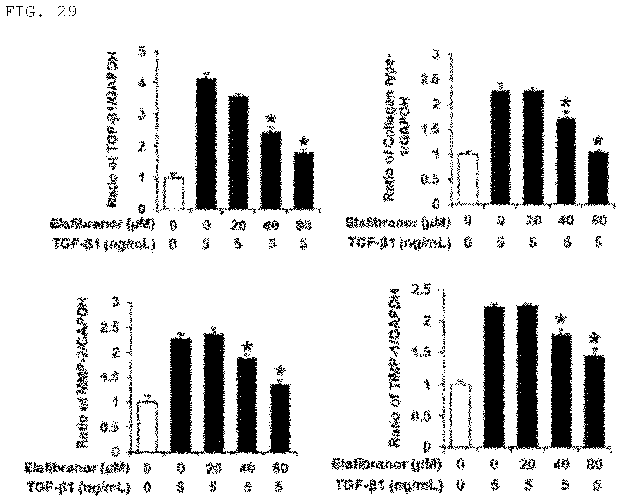

FIG. 29 is a diagram for confirming changes in the expression of liver fibrosis inhibition-related proteins according to a control group. Elafibranor (Genfit) is a new drug candidate for the prevention and treatment of non-alcoholic fatty liver disease.

Specifically, the evaluation was performed by using LX-2 cells, which are hepatic stellate cells. The LX-2 cells treated with 5 ng/ml TGF-β1 were treated with elafibranor at concentrations of 0 µM, 20 µM, 40 µM, and 80 µM in the control group of the present invention, and the cultured for 24 hours under the same conditions. Then, the protein expression ratios of TGF-β1, collagen type-1, MMP-2, and TIMP1 compared to GAPDH were analyzed.

Referring to FIG. 29, it was confirmed that when elafibranor was treated at 40 µM, the expression ratio of TGF-β1, collagen type-1, MMP-2, and TIMP1 proteins compared to GAPDH was decreased.

Accordingly, considering that when JAKI according to an Example of the present invention was treated at a concentration of 20 µM, the expression ratio of MMP-2 and TIMP1 proteins compared to GAPDH was decreased by about 50% or more compared to the untreated group, and when JAKI was treated at a concentration of 40 µM, the expression ratio of TGF-β1, collagen type-1, MMP-2, and TIMP1 proteins compared to GAPDH was significantly decreased, JAKI showed a much better anti-inflammatory and liver fibrosis inhibitory efficacy at lower concentrations compared to elafibranor.

Illustrative Examples of the present invention have been described above, but the present invention is not limited thereto, and those skilled in the art may understand that various changes and modifications are possible within the scope of the concept and range of the claims described below.

## Claims

1. A composition for preventing and treating inflammatory disease and autoimmune diseases and non-alcoholic fatty liver disease, comprising a *Centipeda minima* extract comprising at least one selected from the group consisting of Brevilin A, Arnicolide D, Arnicolide C, and Microhelenin C of the formulas below as an active ingredient.

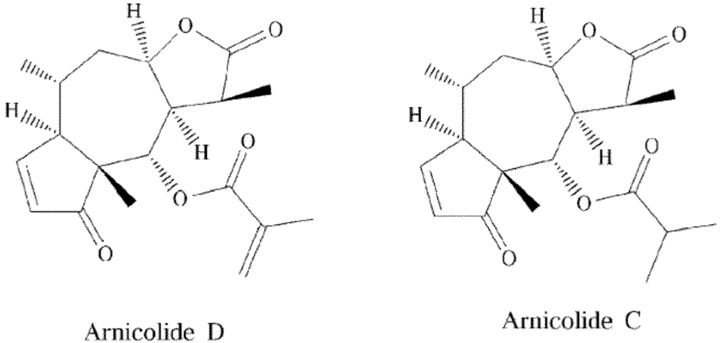

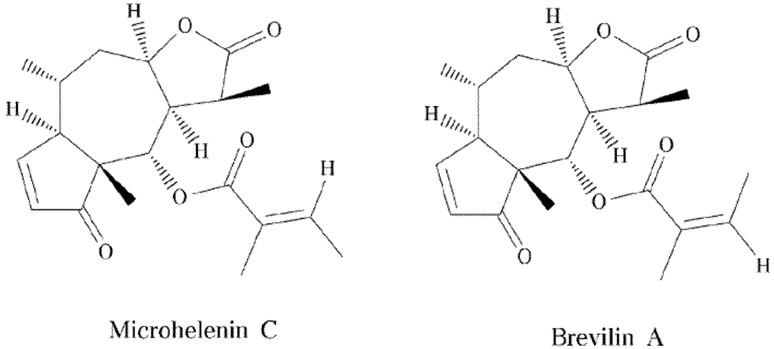

2. The composition for preventing and treating inflammatory disease and autoimmune diseases and non-alcoholic fatty liver disease according to claim 1, wherein the Brevilin A inhibits or suppresses JAK-STAT signaling process; the Arnicolide D, the Arnicolide C, and the Microhelenin C inhibit or suppress cytokine-cytokine receptor interaction, MAPK signaling pathway, and PI3K-AKT signaling pathway; and the *Centipeda minima* extract activates WNT signaling pathway (WNT/β-catenin pathway).

3. The composition for preventing and treating inflammatory disease and autoimmune diseases and non-alcoholic fatty liver disease according to claim 1, wherein the content of the Brevilin A is more than double the total content of the Arnicolide D, the Arnicolide C, and the Microhelenin C.

4. The composition for preventing and treating inflammatory disease and autoimmune diseases and non-alcoholic fatty liver disease according to claim 1, wherein the *Centipeda minima* extract comprises 30 to 90% by weight of the Brevilin A;
0 to 30% by weight of the Arnicolide D; 0 to 30% by weight of the Arnicolide C; and 0 to 15% by weight of the Microhelenin C.

5. The composition for preventing and treating inflammatory disease and autoimmune diseases and non-alcoholic fatty liver disease according to claim 1, wherein the *Centipeda minima* extract is extracted by an extraction method comprising: preparing a primary extraction by extracting a *Centipeda minima* raw material; performing secondary extraction by mixing a phase separation composition comprising a lipophilic solubilizer and water with the primary extraction; and obtaining a non-polar natural substance by separating a top layer of a phase-separated solution.

6. The composition for preventing and treating inflammatory disease and autoimmune diseases and non-alcoholic fatty liver disease according to claim 1, wherein the composition for preventing and treating inflammatory disease and autoimmune diseases and non-alcoholic fatty liver disease further comprises at least one selected from the group consisting of a lipophilic solubilizer, a surfactant, and water.

7. The composition for preventing and treating inflammatory disease and autoimmune diseases and non-alcoholic fatty liver disease according to claim 6, wherein the lipophilic solubilizer further comprises at least one selected from the group consisting of BRIJ 010-SS-(RB), Capryol 90, Capryol PGMC, Gantrez ES-435, Gantrez S-97 BF, Gelucire 43/01, Gelucire 44/14, Gelucire 50/13, Gelucire 48/16, Labrafac CC, Labrafac Lipophile WL1349, Labrafac PG, Labrafil M 1944 CS, Labrafil M 2125 CS, Labrafil M 2130 CS, Lauroglycol 90, Lauroglycol FCC, Monosteol, Peceol, Pharmasolve, Plurol Oleique CC497, Span 20-LQ-(SG), Span 60-PA-(SG), upper Refined Oleic Acid-LQ-(JP), Super Refined Tween 80A-LQ-(MH), Surfadone LP-300, Transcutol HP, Transcutol P, TWEEN 60-SS-(SG), TWEEN 80 HP-LQ-(MH), TWEEN 80-LQ-(SG), Vitamin E TPGS, Miglyol 812, and captax 355.

8. The composition for preventing and treating inflammatory disease and autoimmune diseases and non-alcoholic fatty liver disease according to claim 6, wherein the surfactant comprises at least one selected from the group consisting of Labrasol, Crodex A-PA-(RB), Geleol, Gelot 64, Suppocire AS2X, Suppocire BML, Synperonic PE/F 127, Synperonic PE/L 44, and Tefose 63.

9. The composition for preventing and treating inflammatory disease and autoimmune diseases and non-alcoholic fatty liver disease according to claim 1, wherein the inflammatory disease and autoimmune diseases and non-alcoholic fatty liver disease comprise at least one selected from the group consisting of hair loss, alopecia areata, rheumatoid arthritis, psoriasis, atopy, eczema, seborrheic dermatitis, scleroderma, skin immune hypersensitivity, multiple sclerosis, lupus, Behçet's disease, ankylosing spondylitis, Sjogren's syndrome, Crohn's disease, and inflammatory bowel disease.

10. A preventive and therapeutic agent of inflammatory disease and autoimmune diseases and non-alcoholic fatty liver disease, comprising a composition for preventing and treating inflammatory disease and autoimmune diseases and non-alcoholic fatty liver disease according to any one of claims 1-9.

11. The preventive and therapeutic agent of inflammatory disease and autoimmune diseases and non-alcoholic fatty liver disease according to claim 10, wherein the composition for preventing and treating inflammatory disease and autoimmune diseases and non-alcoholic fatty liver disease comprises 0.1 to 90% by weight of the extract of the *Centipeda minima* extract based on the total weight of the composition.
